(19) Europäisches Patentamt
European Patent Office
Office européen des brevets

(11) Publication number: **0 207 563 B1**

(12) # EUROPEAN PATENT SPECIFICATION

(45) Date of publication of patent specification: **31.07.91**

(51) Int. Cl.[5]: **A01N 43/50**, C07D 233/90, C07D 401/04, C07D 405/04, C07D 409/04

(21) Application number: **86201098.0**

(22) Date of filing: **24.06.86**

(54) **A method for controlling weeds.**

(30) Priority: **01.07.85 GB 8516573**

(43) Date of publication of application:
**07.01.87 Bulletin 87/02**

(45) Publication of the grant of the patent:
**31.07.91 Bulletin 91/31**

(84) Designated Contracting States:
**AT BE CH DE FR GB IT LI LU NL SE**

(56) References cited:
**EP-A- 0 000 373**
**FR-A- 1 184 709**
**FR-A- 1 367 746**
**US-A- 3 485 917**
**US-A- 3 547 942**
**US-A- 3 873 297**

(73) Proprietor: **JANSSEN PHARMACEUTICA N.V.**
**Turnhoutseweg 30**
**B-2340 Beerse(BE)**

(72) Inventor: **Lutz, William R.**
**Mühlestieg 28**
**CH-4125 Riehen(CH)**
Inventor: **van Lommen, Guy Rosalia Eugène**
**Klets 34**
**B-2590-Berlaar(BE)**
Inventor: **van Gestel, Jozef Frans Elisabetha**
**Renier Sniederspad 9**
**B-2350-Vosselaar(BE)**

**EP 0 207 563 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a novel method for controlling weeds, preferably selectively in crops of useful plants. Further, the invention also relates to novel compounds used in said new method; to processes for preparing these compounds and to compositions containing them as active ingredients.

According to the method of the present invention weeds can be controlled by applying thereto or to the locus thereof of a herbicidally effective amount of a 5-imidazolecarboxylic acid derivative of formula

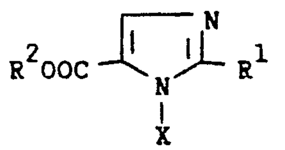

(I)

or a stereochemically isomeric form thereof, or of a salt thereof,
wherein
$R^1$ is hydrogen or mercapto,
$R^2$ is hydrogen, $C_1$-$C_7$alkyl, $C_3$-$C_7$alkenyl, $C_3$-$C_7$alkynyl, $C_1$-$C_7$alkoxy-$C_1$-$C_7$alkyl, aryl-$C_1$-$C_5$alkyl or $C_3$-$C_7$cycloalkyl and
X is 1-indanyl, 1-tetrahydronaphthalenyl, 5-benzocycloheptanyl, 4-tetrahydrobenzothienyl, 4-tetrahydrobenzofuryl, 5-tetrahydroquinolyl, 5-tetrahydroisoquinolyl, 8-tetrahydroquinolyl, 8-tetrahydroisoquinolyl, 9,10-dihydro-9-antracenyl, 9H-fluoren-9-yl, 5-dibenzo[a,d]cycloheptenyl, 5-dibenzo[a,d]cycloheptanyl or 1-dihydronaphthalenyl each unsubstituted or substituted with one to six substituents selected from the group consisting of $C_1$-$C_5$alkyl, mono- and di(aryl) $C_1$-$C_5$alkyl, $C_1$-$C_5$alkoxy, halo, $C_3$-$C_7$alkenyl, amino, nitro, $C_1$-$C_6$alkylcarbonylamino, trifluoromethyl and difluoromethoxy, wherein two geminal substituents together with the carbon atom to which they are attached may form a $C_3$-$C_7$cycloalkyl group; wherein aryl is phenyl optionally substituted with one to three substituents each independently selected from the group consisting of $C_1$-$C_5$alkyl, $C_1$-$C_5$alkyloxy and halo.

Some of the active ingredients of formula (I), methods of preparing them and their pharmaceutical use as antifungal agents are known from US-A-3,485,917. Further, some of these compounds were described as active agents in a method for inhibiting bud growth in US-A-3,873,297. The majority of compounds of formula (I) are novel and have especially been developped to be used in the method of this invention.

Surprisingly, the compounds of formula (I) exhibit strong herbicidal properties, and are therefore useful to control weeds. This property gains importance by the fact that some crops of useful plants are not damaged, or are only slightly harmed at high dosages, when treated with compounds of formula (I). Consequently, the compounds of formula (I) are valuable selective herbicides in crops of useful plants, such as rice and maize. Especially in rice crops a broad range of application rates can be employed, preferably if the rice crops are transplanted rice crops, and if the compounds of formula (I) are applied after transplantation. In maize crops selective herbicidal action is observed both at preemergence and at postemergence treatment.

The active ingredients of formula (I) are usually applied at application rates of 0.01 to 5.0 kg of active ingredient per hectare in order to achieve satisfying results. Sometimes, depending on the evironmental conditions, the application rates may exceed the above designated limitations. However, the preferred application rates are between 0.02 kg and 1.0 kg a.i. per hectare.

As used in the foregoing definitions $C_1$-$C_5$alkyl denotes straight or branch chained saturated hydrocarbon radicals having from 1 to 5 carbon atoms, e.g. methyl, ethyl, n-propyl, isopropyl, the four butyl isomers, the pentyl isomers; $C_1$-$C_7$alkyl includes $C_1$-$C_5$alkyl radicals and the higher homologs thereof having 6 or 7 carbon atoms;
halo is fluoro, chloro, bromo or iodo, with fluoro and chloro being preferred;
$C_3$-$C_7$alkenyl defines straight and branched chained hydrocarbon radicals containing one double bond and having from 3 to 7 carbon atoms such as, for example, allyl, 3-butenyl, 2-butenyl, 2-pentenyl, 3-pentenyl, methallyl, or 3-methyl-2-butenyl, with allyl and methallyl being preferred;
$C_3$-$C_7$alkynyl defines straight and branch chained hydrocarbon radicals containing one triple bond and having from 3 to 7 carbon atoms such as, for example, propargyl, 2-butynyl, 3-butynyl, 2-pentynyl, 3-pentynyl or 4-pentynyl, with propargyl being preferred;
$C_3$-$C_7$cycloalkyl is generic to cyclopropyl, cyclobutyl, cyclopentyl, cyclohexyl and cycloheptyl, with cyclopentyl and cyclohexyl being preferred.

As typical examples of the aryl defined hereinabove, there may be mentioned phenyl, 4-chlorophenyl, 4-fluorophenyl, 2,4-dichlorophenyl, 4-methoxyphenyl, 4-methylphenyl, 3-chlorophenyl, 3,4-dimethoxyphenyl, 2,5-dichlorophenyl, 2,4-dimethoxyphenyl. As examples of aryl-$C_1$-$C_5$-alkyl or diaryl-$C_1$-$C_5$alkyl groups there may be mentioned benzyl, phenylethyl, 4-chlorobenzyl, 4-chlorophenylethyl, benzhydryl, 4-methoxybenzyl, 3-methoxybenzyl, or 4,4'-dichlorobenzhydryl, benzyl and benzhydryl being preferred. $C_1$-$C_5$-alkoxy denotes, for example, methoxy, ethoxy, n-propyloxy, isopropyloxy, the four butyloxy isomers or the pentyloxy isomers; $C_1$-$C_7$alkoxy$C_1$-$C_7$alkyl denotes for example methoxymethyl, methoxyethyl, methoxypropyl, ethoxymethyl, ethoxyethyl, ethoxypropyl, propoxymethyl, propoxyethyl,propoxypropyl, isopropoxymethyl isopropoxyethyl, isopropoxypropyl, 2-methoxypropyl, 2-ethoxypropyl, 2-methoxybutyl, 3-methoxybutyl, 2-ethoxybutyl, or 3-ethoxybutyl.

It is evident that the substituents on the radical X may be attached to different carbon atoms, or two of them may also take geminal positions.

Depending on the nature of the moiety X and/or the group $R^2$ the compounds of formula (I) contain asymmetrical carbon atoms. Unless otherwise mentioned or indicated, the chemical designation of compounds denotes the mixtures of all stereochemically isomeric forms. These mixtures contain all diastereomeres and enantiomeres of the basic molecular structure.

Pure isomeric forms of these compounds can be separated from the mixtures by conventional separation methods. Preferably, if a specific stereochemical form is desired, said compound will be synthesized by stereoselective methods of preparation. These methods will advantageously employ optically active starting materials.

The invention also comprises the use of the salts which the compounds of formula (I) are able to form with organic or inorganic bases such as amines, alkali metal bases and earth alkaline metal bases, or quaternary ammonium bases, or with organic or inorganic acids, such as mineral acids, sulfonic acids, carboxylic acids or phosphorus containing acids.

Examples for salt-forming mineral acids are hydrofluoric acid, hydrochloric acid, hydrobromic acid, hydroiodic acid, sulfuric acid, nitric acid, chloric acid, perchloric acid or phosphoric acid. Prefered salt-forming sulfonic acids are toluenesulfonic acid, benzenesulfonic acid, methanesulfonic acid or trifluoromethanesulfonic acid. Preferred salt-forming carboxylic acids are acetic acid, trifluoroacetic acid, benzoic acid, chloroacetic acid, phthalic acid, maleic acid, malonic acid and citric acid. Phosphorous containing acids are the various phosphonous acids, phosphonic acids and phosphinic acids.

Preferred salt-forming alkali metal hydroxides and earth alkaline metal hydroxides are the hydroxides of lithium, sodium, potassium, magnesium or calcium, most preferably those of sodium or potassium. Examples of suitable salt-forming amines are primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline. Preferred amines are ethylamine, propylamine, diethylamine or triethylamine, with isopropylamine, diethanolamine and 1,4-diazabicyclo[2.2.2]octane being most preferred. Examples of quaternary ammonium bases are, in general, the cations of haloammonium salts, e.g. the tetramethylammonium cation, the trimethylbenzylammonium cation, the triethylbenzylammonium cation, the tetraethylammonium cation, the trimethylethylammonium cation, and also the ammonium cation.

A preferred subgroup of active ingredients used in the method of this invention are those compounds of formula (I), wherein

$R^2$ is hydrogen, $C_1$-$C_7$alkyl, $C_3$-$C_7$alkenyl, $C_3$-$C_7$alkynyl or $C_3$-$C_7$cycloalkyl, and

X is 1-indanyl, 1-tetrahydronaphthalenyl or 5-benzocycloheptanyl, each unsubstituted or substituted with one to six substituents independently selected from $C_1$-$C_5$alkyl, $C_1$-$C_5$alkoxy, halo, trifluoromethyl or difluoromethoxy.

Within this preferred group, those compounds are particularly preferred, wherein X is a radical of formula:

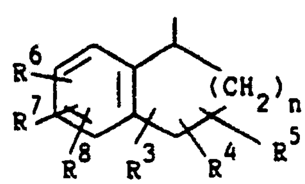

(a)

wherein

$R^3$, $R^4$ and $R^5$ are each independently hydrogen or $C_1$-$C_5$alkyl, $R^6$, $R^7$ and $R^8$ are each independently hydrogen or $C_1$-$C_5$alkyl, $C_1$-$C_5$alkoxy, halo, $CF_3$ or $OCHF_2$, and
n is 1 or 2 or 3.

Within this group of particularly preferred compounds special preference is put to compounds wherein either

a) n is two or
b) n is one or
c) $R^3$, $R^4$ and $R^5$ are each independently hydrogen or methyl or
d) $R^6$, $R^7$ and $R^8$ are each independently hydrogen, methyl, methoxy, fluoro, chloro or bromo or
e) $R^2$ is $C_1$-$C_4$alkyl.

From subgroup e) those compounds are preferred wherein $R^2$ is methyl.

A more particularly preferred subgroup comprises those compounds wherein n is two, $R^3$, $R^4$ and $R^5$ are each independently hydrogen or methyl, $R^6$, $R^7$ and $R^8$ are each independently hydrogen, methyl, methoxy, fluoro, chloro or bromo, and $R^2$ is $C_1$-$C_4$alkyl. Among this group again those compounds are preferred, wherein $R^2$ is methyl.

The same preference is given to compounds wherein n is one, $R^3$, $R^4$ and $R^5$ are each independently hydrogen or methyl, $R^6$, $R^7$ and $R^8$ are each independently hydrogen, methyl, methoxy, fluoro, chloro or bromo, and $R^2$ is $C_1$-$C_4$alkyl.

The most preferred compounds which can be used in the method according to this invention are selected among
1-(1,2,3,4-tetrahydronaphthalen-1-yl)-5-imidazolecarboxylic acid methyl ester,
1-(2-methyl-1,2,3,4-tetrahydronaphthalen-1-yl)-5-imidazolecarboxylic acid methyl ester,
1-(2,2-dimethyl-1,2,3,4-tetrahydronaphthalen-1-yl)-5-imidazolecarboxylic acid methyl ester,
1-(5,7-dimethyl-1,2,3,4-tetrahydronaphthalen-1-yl)-2-mercapto-5-imidazolecarboxylic acid methyl ester,
1-(1,2,3,4-tetrahydronaphthalen-1-yl)-2-mercapto-5-imidazolecarboxylic acid methyl ester,
1-(2,2-dimethylindan-1-yl)-2-mercapto-5-imidazolecarboxylic acid methyl ester, and
1-(2,2-dimethylindan-1-yl)-5-imidazolecarboxylic acid methyl ester.

As mentioned above most of the active ingredients of formula (I) are novel compounds and have especially been developped to be used as active substances in the novel method for controlling weeds according to the present invention. These compounds therefore constitute a further aspect of the invention. The said novel compounds can be represented by the formula

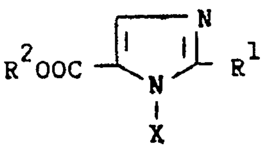

(I')

wherein, $R^1$, $R^2$ and X have the previously defined meaning, provided that X is other than unsubstituted 1-indanyl, or unsubstituted 1-tetrahydronaphthalenyl.

Preferred novel compounds are those wherein X is a radical of formula (a) as defined hereinabove, wherein n is one, $R^3$, $R^4$ and $R^5$ are each independently hydrogen or methyl, $R^6$, $R^7$ and $R^8$ are each independently hydrogen, methyl, methoxy, fluoro, chloro or bromo, and $R^2$ is $C_1$-$C_4$alkyl.

The most preferred novel compounds are:
1-( 2-methyl-1,2,3,4-tetrahydronaphthalen-1-yl)-5-imidazolecarboxylicacid methyl ester,
1-(2,2-dimethyl-1,2,3,4-tetrahydronaphthalen-1-yl)-5-imidazolecarboxylic acid methyl ester,
1-(5,7-dimethyl-1,2,3,4-tetrahydronaphthalen-1-yl)-5-imidazolecarboxylic acid methyl ester,
(2,2-dimethylindan-1-yl)-2-mercapto-5-imidazolecarboxylic acid methyl ester, and
1-(2,2-dimethylindan-1-yl)-5-imidazolecarboxylic acid methyl ester.

The preparation of the compounds of formula (I), both the novel ones and the known ones, is generally carried out by the following methods.

The compounds of formula (I) can be obtained by condensing a compound of formula

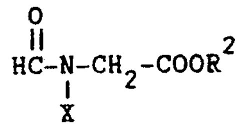

(II)

wherein $R^2$ and X are as defined hereinabove, with a $C_1$-$C_4$alkyl ester of formic acid in the presence of suitable base such as, for example, an alkali metal alkoxide or hydride, e.g. sodium methoxide, potassium ethoxide, sodium hydride, lithium hydride, and the like, in a reaction-inert solvent; and treating the resultant intermediate of formula

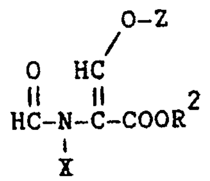

(III)

wherein $R^2$ and X are as defined hereinabove and Z is an alkali metal atom,

a) with an alkali metal isothiocyanate in the presence of an acid, thus obtaining a 2-mercaptoimidazole of formula

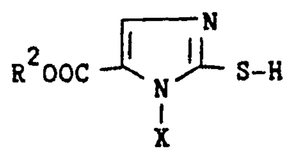

(Ia)

wherein $R^2$ and X are as defined hereinabove, which optionally is converted into a compound of the formula

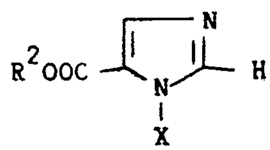

(Ib)

by reacting the starting compound with sodium nitrite in the presence of nitric acid in an aqueous medium; or with Raney-nickel in the presence of a lower aliphatic alcohol, preferably ethanol, at a temperature between 40° C and 80° C; or also by treating the starting compounds with an aqueous hydrogen peroxide solution preferably in the presence of a carboxylic acid, e.g. acetic acid; or

b) with a carboxylic acid amide of 1 to 3 carbon atoms, preferably formamide, in the presence of an acid at a temperature between 50° C and 250° C, preferably between 120° C and 170° C; or

c) with an excess of ammonium carbonate or hydrogen carbonate in a suitable solvent, which may be a reaction-inert solvent or an acid, at a temperature between 20° C and 200° C, preferably between 25° C and the reflux temperature of the reaction mixture.

In the afore-mentioned processes reaction-inert solvents are, for example, aromatic hydrocarbons such as benzene, toluene or xylene; ethers such as, for example, diethylether, tetrahydrofuran or dioxane; or other aprotic organic solvents. For the cyclization-reaction of the imidazole ring structure, strong mineral acids such as hydrohalic acids, e.g. hydrochloric acid, are most conveniently employed. In the ring-forming variant c) also other acids, e.g. acetic acid, can be used.

In this reaction an excess of acid of 5 to 50, preferably of 15 to 40 times the required molar amount is most preferably used. The excess of ammonium salt in this process is 2 to 50, preferably 10 to 30 times the required molar amount.

The substituent $R^2$ on the carboxylic acid group may be transformed to other substituents encompassed by the definition of $R^2$ by convenient reactions known in the art for the modification of carboxylic acid

functions, e.g. by hydrolysis and esterification and/or transesterification.

If the synthesis of sterochemically pure isomers is intended, stereoselective reaction steps and conditions are recommended. On the other hand conventional methods of separation can be used for obtaining pure isomers from a mixture of stereochemical isomers.

The starting materials for the preparation of the novel compounds of formula (I) are known, or they can be obtained by known synthesis procedures.

For examples, the compounds of formula (II) can be obtained by reacting a glycine ester of formula

$Z\text{-}NH\text{-}CH_2\text{-}COOR^2$ (IV)

wherein $R^2$ and Z are as defined hereinabove, with formic acid in the presence of acetic anhydride. In turn, the compounds of formula (IV) can be prepared by reacting an amine of formula

$Z\text{-}NH_2$ (V)

wherein Z is as defined under formula (I), with a $\alpha$-bromoacetic acid ester of formula

$Br\text{-}CH_2\text{-}COOR^2$ (VI)

wherein $R^2$ is as defined under formula (I), in the presence of an acid-binding agent, such as sodium carbonate.

The compounds of formula (I) are stable compounds and no precautionary measures are required for handling them.

When used at the indicated rates of application, the compounds of formula (I) have good selective herbicidal properties which make them most suitable for use in crops of useful plants, preferably in maize and in rice. In some cases damage is also caused to weeds which up to now have only been controlled up to now with total herbicides.

At higher rates of application, all tested plants are so severely damaged in their development that they die.

The invention also relates to herbicidal compositions which contain as an active ingredient a novel compound of formula (I') as defined hereinabove. Preferred methods of controlling weeds make use of the novel compounds.

In the method for controlling weeds according to the invention the compounds of formula (I) are used in unmodified form or, preferably, together with the adjuvants conventionally employed in the art of formulation, and are therefore formulated in known manner to emulsifiable concentrates, directly sprayable or dilutable solutions, dilute emulsions, wettable powders, soluble powders, dusts, granulates, and also encapsulations in e.g. polymer substances. As with the nature of the compositions, the methods of application, such as spraying, atomising, dusting, scattering or pouring, are chosen in accordance with the intended objectives and the prevailing circumstances.

The formulations, i.e the compositions, preparations or mixtures containing the compound (active ingredient) of formula (I) and, where appropriate, a solid or liquid adjuvant, are prepared in a known manner, e.g. by homogeneously mixing and/or grinding the active ingredients with extenders, e.g. solvents, solid carriers and, where appropriate, surface-active compounds (surfactants).

Suitable solvents are: aromatic hydrocarbons, preferably the fractions containing 8 to 12 carbon atoms, e.g. xylene mixtures or substituted naphthalenes, phthalates such as dibutyl phthalate or dioctyl phthalate, aliphatic hydrocarbons such as cyclohexane or paraffins, alcohols and glycols and their ethers and esters, such as ethanol, ethylene glycol, ethylene glycol monomethyl or monoethyl ether, ketones such as cyclohexanone, strongly polar solvents such as N-methyl-2-pyrrolidone, dimethylsulfoxide or dimethylformamide, as well as vegetable oils or epoxidised vegetable oils such as epoxidised coconut oil or soybean oil; or water.

The solid carriers used e.g. for dusts and dispersible powders are normally natural mineral fillers such as calcite, talcum, kaolin, montmorillonite or attapulgite. In order to improve the physical properties it is also possible to add highly dispersed silicic acid or highly dispersed absorbent polymers. Suitable granulated adsorptive carriers are porous types, for example pumice, broken brick, sepiolite or bentonite; and suitable nonsorbent carriers are materials such as calcite or sand. In addition, a great number of pregranulated materials of inorganic or organic nature can be used, e.g. especially dolomite or pulverised plant residues.

Depending on the nature of the compound of formula(I) to be formulated, suitable surface-active compounds are nonionic, cationic and/or anionic surfactants having good emulsifying, dispersing and

wetting properties. The term "surfactants" will also be understood as comprising mixtures of surfactants.

Suitable anionic surfactants can be both water-soluble soaps and water-soluble synthetic surface-active compounds.

Suitable soaps are the alkali metal salts, earth alkaline metal salts or unsubstituted or substituted ammonium salts of higher fatty acids ($C_{10}$-$C_{22}$), e.g. the sodium or potassium salts of oleic or stearic acid, or of natural fatty acid mixtures which can be obtained e.g. from coconut oil or tallow oil. Mention may also be made of fatty acid methyltaurin salts.

More frequently, however, so-called synthetic surfactants are used, especially fatty sulfonates, fatty sulfates, sulfonated benzimidazole derivatives or alkylarylsulfonates.

The fatty sulfonates or sulfates are usually in the form of alkali metal salts, alkaline earth metal salts or unsubstitued or substituted ammonium salts and contain a $C_8$-$C_{22}$alkyl radical which also includes the alkyl moiety of acyl radicals, e.g. the sodium or calcium salt of lignosulfonic acid, of dodecylsulfate or of a mixture of fatty alcohol sulfates obtained from natural fatty acids. These compounds also comprise the salts of sulfuric acid esters and sulfonic acids of fatty alcohol/ethylene oxide adducts. The sulfonated benzimidazole derivatives preferably contain 2 sulfonic acid groups and one fatty acid radical containing 8 to 22 carbon atoms. Examples of alkylarylsulfonates are the sodium, calcium or triethylanolamine salts of dodecylbenzene sulfonic acid, dibutylnaphthalenesulfonic acid, or of a naphthalenesulfonic acid/formaldehyde condensation product.

Also suitable are corresponding phosphates, e.g. salts of the phosphoric acid ester of an adduct of p-nonylphenol with 4 to 14 moles of ethylene oxide, or phospholipids.

Non-ionic surfactants are preferably polyglycol ether derivatives of aliphatic or cycloaliphatic alcohols, or saturated or unsaturated fatty acids and alkylphenols, said derivatives containing 3 to 10 glycol ether groups and 8 to 20 carbon atoms in the (aliphatic) hydrocarbon moiety and 6 to 18 carbon atoms in the alkyl moiety of the alkylphenols.

Further suitable non-ionic surfactants are the water-soluble adducts of polyethylene oxide with polypropylene glycol, ethylenediaminopolypropylene glycol and alkylpolypropylene glycol containing 1 to 10 carbon atoms in the alkyl chain, which adducts contain 20 to 250 ethylene glycol ether groups and 10 to 100 propylene glycol ether groups. These compounds usually contain 1 to 5 ethylene glycol units per propylene glycol unit.

Representative examples of non-ionic surfactants are nonylphenolpolyethoxyethanols,castor oil polyglycol ethers, polypropylene/polyethylene oxide adducts, tributylphenoxypolyethoxyethanol, polyethylene glycol and octylphenoxypolyethoxyethanol.

Fatty acid esters of polyoxyethylene sorbitan, such as polyoxyethylene sorbitan trioleate, are also suitable non-ionic surfactants.

Cationic surfactants are preferably quaternary ammonium salts which contain, as N-substituent, at least one $C_8$-$C_{22}$alkyl radical and, as further substituents, unsubstituted or halogenated lower alkyl, benzyl or hydroxy-lower alkyl radicals. The salts are preferably in the form of halides, methylsulfates or ethylsulfates, e.g. stearyltrimethylammonium chloride or benzyldi(2-chloroethyl)ethylammonium bromide.

The surfactants customarily employed in the art of formulation are described e.g. in the following publications:
"McCutcheon's Detergents and Emulsifiers Annual",
MC Publishing Corp., Ridgewood, New Jersey, 1981; H. Stache,
"Tensid-Taschenbuch", 2nd Edition, C. Hanser Verlag,
Munich & Vienna, 1981; M. and J. Ash, "Encyclopedia of Surfactants",
Vol. I-III, Chemical Publishing Co., New York, 1980-81.

The herbicidal compositions which are preferably employed in the process of the invention usually contain 0.1 to 95 %, preferably 0.1 to 80 %, of a compound of formula(I),
1 to 99.9 %, of a solid or liquid adjuvant, and 0 to 25 %, preferably 0.1 to 25 %, of a surfactant.

Preferred formulations are composed in particular of the following constituents (% = percentage by weight):

<u>Emulsifiable concentrates</u>

| | |
|---|---|
| active ingredient: | 1 to 20 %, preferably 5 to 10 % |
| surfactant: | 5 to 30 %, preferably 10 to 20 % |
| liquid carrier: | 50 to 94 %, preferably 70 to 85 % |

<u>Dusts</u>

| | |
|---|---|
| active ingredient: | 0.1 to 10 %, preferably 0.1 to 1 % |
| solid carrier: | 99.9 to 90 %, preferably 99.9 to 99 % |

<u>Suspension concentrates</u>

| | |
|---|---|
| active ingredient: | 5 to 75 %, preferably 10 to 50 % |
| water: | 94 to 25 %, preferably 88 to 30 % |
| surfactant: | 1 to 40 %, preferably 2 to 30 % |

<u>Wettable powders</u>

| | |
|---|---|
| active ingredient: | 0.5 to 90 %, preferably 1 to 80 % |
| surfactant: | 0.5 to 20 %, preferably 1 to 15 % |
| solid carrier: | 5 to 95 %, preferably 15 to 90 % |

<u>Granulates</u>

| | |
|---|---|
| active ingredient: | 0.5 to 30 %, preferably 3 to 15 % |
| solid carrier: | 99.5 to 70 %, preferably 97 to 85 %. |

Some of the compounds of the formula (I) which are used as active ingredients in the method for controlling weeds in accordance with the invention are listed in the following tables with the purpose of illustrating the invention and not of limiting it thereto.

Table 1:

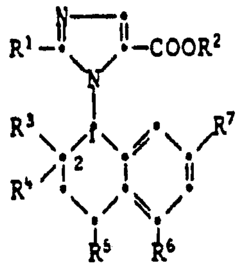

| Comp. No. | $R^1$ | $R^2$ | $R^3$ | $R^4$ | $R^5$ | $R^6$ | $R^7$ | physical data |
|---|---|---|---|---|---|---|---|---|
| 1.01 | H | $CH_3$ | H | H | H | H | H | $\cdot HNO_3$/m.p. 149-151.5°C |
| 1.02 | H | $CH_3$ | H | H | H | H | H | m.p.63°C |
| 1.03 | H | $CH_3$ | $CH_3$ | $CH_3$ | H | H | H | m.p.100-101°C |
| 1.04 | H | $CH_3$ | H | H | $CH_3$ | H | H | $\cdot HNO_3$/ m.p.117-118°C |
| 1.05 | H | $CH_3$ | H | H | H | H | $OCH_3$ | resin |
| 1.06 | H | $CH_3$ | H | H | H | $CH_3$ | $CH_3$ | $\cdot HNO_3$/m.p.136°C (decomp.) |
| 1.07 | H | $CH_3$ | H | H | H | $OCH_3$ | H | $\cdot HNO_3$/ m.p.139-140°C |
| 1.08 | H | $CH_3$ | $CH_3$ | $CH_3$ | H | H | H | $\cdot HNO_3$/m.p.160°C (decomp.) |
| 1.09 | H | $CH_3$ | H | H | H | $CH_3$ | $CH_3$ | resin |
| 1.10 | H | $CH_3$ | H | H | H | H | F | $\cdot HNO_3$/m.p.161°C |
| 1.11 | SH | $CH_3$ | H | H | $CH_3$ | H | H | m.p.171-172°C |
| 1.12 | SH | $CH_3$ | $CH_3$ | H | H | H | H | m.p.154-155°C |
| 1.13 | SH | $CH_3$ | H | H | H | H | $OCH_3$ | m.p.184-186°C |
| 1.14 | SH | $CH_3$ | H | H | H | $CH_3$ | $CH_3$ | m.p.190-190.5°C |
| 1.15 | SH | $CH_3$ | H | H | H | $OCH_3$ | H | m.p.170-171°C |
| 1.16 | SH | $CH_3$ | H | H | H | H | H | m.p.157-158°C |
| 1.17 | SH | $CH_3$ | $CH_3$ | $CH_3$ | H | H | H | m.p.171-172°C |
| 1.18 | H | $C_2H_5$ | H | H | H | H | H | $.HNO_3$/m.p. 135-136°C |
| 1.19 | H | $C_3H_7$-n | H | H | H | H | H | $.HNO_3$/m.p. 109-110°C |
| 1.20 | H | $C_2H_5$ | $CH_3$ | H | H | H | H | |
| 1.21 | H | $CH_3$ | H | H | $OCH_3$ | H | H | |
| 1.22 | H | $CH_3$ | H | H | H | $OCH_3$ | $OCH_3$ | |

Table 1: (Continuation)

| Comp. No. | $R^1$ | $R^2$ | $R^3$ | $R^4$ | $R^5$ | $R^6$ | $R^7$ | physical data |
|---|---|---|---|---|---|---|---|---|
| 1.23 | H | $C_2H_5$ | H | H | H | $CH_3$ | $CH_3$ | |
| 1.24 | H | $C_3H_7$-i | H | H | H | $OCH_3$ | H | |
| 1.25 | H | $CH_3$ | $CH_3$ | $CH_3$ | H | $CH_3$ | H | |
| 1.26 | H | $C_3H_7$-n | H | H | H | $CH_3$ | $CH_3$ | |
| 1.27 | H | $C_2H_5$ | H | H | H | H | F | |
| 1.28 | SH | $C_2H_5$ | H | H | $CH_3$ | H | H | |
| 1.29 | SH | $C_2H_5$ | $CH_3$ | H | H | H | H | |
| 1.30 | SH | $CH_3$ | H | H | H | $OCH_3$ | $OCH_3$ | |
| 1.31 | SH | $C_2H_5$ | H | H | H | $CH_3$ | $CH_3$ | |
| 1.32 | SH | $C_3H_7$-i | H | H | H | $OCH_3$ | H | |
| 1.33 | SH | $C_3H_7$-n | H | H | H | H | H | |
| 1.34 | SH | $CH_3$ | $CH_3$ | $CH_3$ | H | $CH_3$ | H | |
| 1.35 | H | $C_6H_{11}$-cycl. | H | H | H | H | H | .$HNO_3$/m.p. 144-145°C |
| 1.36 | SH | $C_5H_9$-cycl. | H | H | H | H | H | |
| 1.37 | H | $C_3H_9$-cycl. | $CH_3$ | $CH_3$ | H | H | H | |
| 1.38 | H | $CH_3$ | H | H | H | Cl | Cl | |
| 1.39 | SH | $C_2H_5$ | H | H | H | Cl | Cl | |
| 1.40 | SH | H | H | H | H | H | H | |
| 1.41 | H | H | H | H | H | H | H | m.p.206.5-210°C |
| 1.42 | H | $C_3H_7$-i | H | H | H | H | H | .$HNO_3$/m.p.138-140°C |
| 1.43 | H | $C_7H_{15}$-n | H | H | H | H | H | |
| 1.44 | SH | $CH_3$ | H | H | H | H | F | m.p.205°C |
| 1.45 | H | $CH_3$ | H | H | H | H | F | m.p.161°C |
| 1.46 | SH | $CH_3$ | H | H | H | H | Br | m.p.206°C |
| 1.47 | H | $CH_3$ | H | H | H | H | Br | m.p.172°C |
| 1.48 | SH | $CH_3$ | H | H | H | H | Cl | m.p.218°C |
| 1.49 | H | $CH_3$ | H | H | H | H | Cl | m.p.131°C |
| 1.50 | SH | $CH_3$ | H | H | H | H | H | m.p. 149-150°C |

Table 1: (Continuation)

| Comp. No. | $R^1$ | $R^2$ | $R^3$ | $R^4$ | $R^5$ | $R^6$ | $R^7$ | physical data |
|---|---|---|---|---|---|---|---|---|
| 1.51 | H | $CH_3$ | H | H | H | H | H | .$(COOH)_2$/m.p. 136-138.5°C |
| 1.52 | H | $CH_2-CH=CH_2$ | H | H | H | H | H | .$HNO_3$/m.p. 103-104°C |
| 1.53 | H | $C_4H_9$-n | H | H | H | H | H | .$HNO_3$/m.p. 126-127°C |
| 1.54 | H | $C_5H_{11}$-n | H | H | H | H | H | .$HNO_3$/m.p. 102-103.5°C |
| 1.55 | H | $(CH_3)_2CH$<br>$CH_2-$ | H | H | H | H | H | .$HNO_3$/m.p. 139-140°C |
| 1.56 | H | $C_2H_5$ | H | H | H | H | H | .$HNO_3$/m.p. 91-92.5°C |
| 1.57 | H | $(CH_3)_2CH$<br>$CH_2$<br>$H_3C-CH-$ | H | H | H | H | H | .$HNO_3$/m.p.133.5-136°C |
| 1.58 | H | $CH_3$ | $CH_3$ | H | H | H | H | .$HNO_3$ |
| 1.59 | H | $CH_3$ | $CH_3$ | H | H | H | H | m.p.90-91°C |
| 1.60 | H | $C_3H_7$-i | $CH_3$ | $CH_3$ | H | H | H | resin |
| 1.61 | H | $C_2H_5$ | $CH_3$ | $CH_3$ | H | H | H | m.p.74-75°C |
| 1.62 | H | $C_4H_9$-n | $CH_3$ | $CH_3$ | H | H | H | m.p.64.5-66.5°C |
| 1.63 | H | $C_5H_{11}$-n | $CH_3$ | $CH_3$ | H | H | H | m.p.72-75°C |
| 1.64 | H | $CH_3$ | $CH_3$ | $CH_3$ | H | H | $OCH_3$ | •$HNO_3$/m.p.163°C (decomp.) |
| 1.65 | H | $CH_3$ | $CH_3$ | $CH_3$ | H | H | $OCH_3$ | resin |
| 1.66 | H | $CH_3$ | $CH_3$ | $CH_3$ | H | $CH_3$ | $CH_3$ | •$HNO_3$/m.p.163°C (decomp.) |
| 1.67 | H | $CH_3$ | $CH_3$ | $CH_3$ | H | $CH_3$ | $CH_3$ | m.p.133-134°C |
| 1.68 | SH | $CH_3$ | $CH_3$ | $CH_3$ | H | $CH_3$ | $CH_3$ | m.p.200-201°C |
| 1.69 | SH | $CH_3$ | $CH_3$ | $CH_3$ | H | H | $OCH_3$ | m.p.166-167°C |
| 1.70 | H | H | $CH_3$ | $CH_3$ | H | H | H | m.p.238°C (decomp.) |
| 1.71 | H | $CH_3$ | $CH_3$ | $CH_3$ | H | H | H | •$CH_3-SO_3H$/ m.p.141-151°C |
| 1.72 | H | $CH_3$ | $CH_3$ | $CH_3$ | H | H | H | •HBr/m.p.146°C (decomp.) |
| 1.73 | H | $CH_3$ | H | H | H | H | H | •HBr/m.p.145°C (decomp.) |

**Table 1:** (Continuation)

| Comp. No. | $R^1$ | $R^2$ | $R^3$ | $R^4$ | $R^5$ | $R^6$ | $R^7$ | physical data |
|---|---|---|---|---|---|---|---|---|
| 1.74 | H | $CH_3$ | H | H | H | H | H | 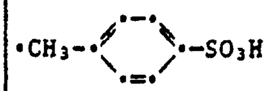 /m.p.178-179°C |
| 1.75 | H | $CH_3$ | $CH_3$ | $CH_3$ | H | H | H | •HCl/m.p.158°C (decomp.) |
| 1.76 | H | $CH_3$ | $CH_3$ | $CH_3$ | H | H | H | $\cdot CF_3COOH$/ m.p.92-93°C |
| 1.77 | H | $Na^{\oplus}$ | H | H | H | H | H | |
| 1.78 | H | $K^{\oplus}$ | H | H | H | H | H | |
| 1.79 | H | $NH_4^{\oplus}$ | H | H | H | H | H | |
| 1.80 | H | $C_4H_9$-n | H | H | H | H | H | resin |
| 1.81 | H | $C_3H_7$-n | H | H | H | H | H | m.p.60-63°C |
| 1.82 | H | $C_6H_{11}$-cycl. | H | H | H | H | H | 86-88°C |
| 1.83 | H | $CH_2-CH_2-OCH_3$ | H | H | H | H | H | 61-63°C |
| 1.84 | H | $CH_2-C\equiv CH$ | H | H | H | H | H | |
| 1.85 | H | $CH_2-CH=CH_2$ | H | H | H | H | H | |
| 1.86 | H | benzyl | H | H | H | H | H | $.HNO_3$/mp. 133.5-134.5°C |
| 1.87 | H | $CH_3$ | $C_2H_5$ | H | H | H | H | $\cdot HNO_3$ |
| 1.88 | H | $CH_3$ | $C_2H_5$ | H | H | H | H | |
| 1.89 | SH | $CH_3$ | $C_2H_5$ | H | H | H | H | |
| 1.90 | H | $CH_3$ | $C_3H_7$-i | H | H | H | H | $\cdot HNO_3$ |
| 1.91 | H | $CH_3$ | $C_3H_7$-i | H | H | H | H | |
| 1.92 | SH | $CH_3$ | $C_3H_7$-i | H | H | H | H | |
| 1.93 | H | $CH_3$ | $C_2H_5$ | $C_2H_5$ | H | H | H | $\cdot HNO_3$ |
| 1.94 | H | $CH_3$ | $C_2H_5$ | $C_2H_5$ | H | H | H | |
| 1.95 | SH | $CH_3$ | $C_2H_5$ | $C_2H_5$ | H | H | H | |
| 1.96 | H | $C_2H_5$ | H | H | H | H | H | resin |
| 1.97 | H | $C_3H_7$-i | H | H | H | H | H | m.p.77-79°C |
| 1.98 | H | $C_6H_{11}$-cycl. | $CH_3$ | H | H | H | H | |
| 1.99 | SH | $CH_3$ | benzyl | H | H | H | H | |
| 1.100 | H | $CH_3$ | benzyl | H | H | H | H | $\cdot HNO_3$ |
| 1.101 | H | $CH_3$ | benzyl | H | H | H | H | |
| 1.102 | H | $C_5H_9$-cycl. | H | H | H | H | H | resin |

**Table 1:** (Continuation)

| Comp. No. | $R^1$ | $R^2$ | $R^3$ | $R^4$ | $R^5$ | $R^6$ | $R^7$ | physical data |
|---|---|---|---|---|---|---|---|---|
| 1.103 | H | $CH_3$ | $C_3H_7$-n | H | H | H | H | |
| 1.104 | SH | $CH_3$ | $C_3H_7$-n | H | H | H | H | |
| 1.105 | H | $CH_3$ | $C_4H_9$-n | H | H | H | H | |
| 1.106 | SH | $CH_3$ | $C_4H_9$-n | H | H | H | H | |
| 1.107 | H | $CH_3$ | $C_5H_{11}$-n | H | H | H | H | |
| 1.108 | SH | $CH_3$ | $C_5H_{11}$-n | H | H | H | H | |
| 1.109 | H | $CH_3$ | H | H | H | $NO_2$ | H | |
| 1.110 | H | $CH_3$ | H | H | H | H | $NO_2$ | |
| 1.111 | H | $CH_3$ | $CH_3$ | $CH_3$ | H | $NO_2$ | H | |
| 1.112 | H | $CH_3$ | $CH_3$ | $CH_3$ | H | H | $NO_2$ | |
| 1.113 | H | $CH_3$ | H | H | H | $NH_2$ | H | |
| 1.114 | H | $CH_3$ | H | H | H | H | $NH_2$ | |
| 1.115 | H | $CH_3$ | H | H | H | $-NHCOCH_3$ | H | |
| 1.116 | H | $CH_3$ | H | H | H | H | $-NHCOCH_3$ | |
| 1.117 | H | $CH_3$ | $CH_3$ | $CH_3$ | H | $NH_2$ | H | |
| 1.118 | H | $CH_3$ | $CH_3$ | $CH_3$ | H | H | $NH_2$ | |
| 1.119 | H | $CH_3$ | $CH_3$ | $CH_3$ | H | $-NHCOCH_3$ | H | |
| 1.120 | H | $CH_3$ | $CH_3$ | $CH_3$ | H | H | $-NHCOCH_3$ | |
| 1.121 | H | $CH_3$ | H | H | H | H | $CF_3$ | |
| 1.122 | H | $CH_3$ | H | H | H | H | $OCHF_3$ | |
| 1.123 | H | $CH_3$ | $-CH(C_6H_5)_2$ | H | H | H | H | |
| 1.124 | SH | $CH_3$ | $CH_3$ | H | H | H | H | 1,2-cis |
| 1.125 | H | $CH_3$ | $CH_3$ | H | H | H | H | 1,2-cis |
| 1.126 | SH | $CH_3$ | $CH_3$ | H | H | H | H | 1,2-trans |
| 1.127 | H | $CH_3$ | $CH_3$ | H | H | H | H | 1,2-trans |

Table 1: (Continuation)

| Comp. No. | $R^1$ | $R^2$ | $R^3$ | $R^4$ | $R^5$ | $R^6$ | $R^7$ | physical date |
|---|---|---|---|---|---|---|---|---|
| 1.128 | H | $CH_2-CH_2-OCH_3$ | H | H | H | H | H | $.HNO_3$/mp. 115-117°C |
| 1.129 | H | $C_2H_4-OC_2H_5$ | H | H | H | H | H | $.HNO_3$/mp. 117-118°C |
| 1.130 | SH | $C_2H_5$ | H | H | H | H | H | $.HNO_3$/mp. 155.5-157°C |
| 1.131 | H | $CH_3$ | $CH_3$ | $CH_3$ | H | H | F | |
| 1.132 | SH | $CH_3$ | $CH_3$ | $CH_3$ | H | H | F | |
| 1.133 | H | $CH_3$ | $CH_3$ | $CH_3$ | H | H | Cl | |
| 1.134 | SH | $CH_3$ | $CH_3$ | $CH_3$ | H | H | Cl | |
| 1.135 | H | $CH_3$ | $CH_3$ | $CH_3$ | H | H | Br | |
| 1.136 | SH | $CH_3$ | $CH_3$ | $CH_3$ | H | H | Br | |
| 1.137 | SH | $CH_3$ | $-CH(C_6H_5)_2$ | H | H | H | H | |
| 1.138 | SH | $CH_3$ | $-CH_2-CH=CH_2$ | H | H | H | H | |
| 1.139 | H | $CH_3$ | $-CH_2-CH=CH_2$ | H | H | H | H | |
| 1.140 | SH | $CH_3$ | $CH_3$ | $C_2H_5$ | H | H | H | |
| 1.141 | H | $CH_3$ | $CH_3$ | $C_2H_5$ | H | H | H | |
| 1.142 | SH | H | $CH_3$ | $CH_3$ | H | H | H | |
| 1.143 | H | H | $CH_3$ | $CH_3$ | H | H | H | |
| 1.144 | H | H | H | H | H | F | H | $H_2O$/mp. 130°C |
| 1.145 | SH | H | H | H | H | F | H | |
| 1.146 | H | H | H | H | H | Cl | H | |
| 1.147 | SH | H | H | H | H | Cl | H | |
| 1.148 | H | H | H | H | H | Br | H | |
| 1.149 | SH | H | H | H | H | Br | H | |
| 1.150 | H | H | $CH_3$ | $CH_3$ | H | F | H | |
| 1.151 | H | H | $CH_3$ | $CH_3$ | H | Cl | H | |
| 1.152 | H | H | $CH_3$ | $CH_3$ | H | $OCH_3$ | H | |
| 1.153 | H | H | H | H | H | $OCH_3$ | H | |

**Table 1**: (Continuation)

| Comp. No. | $R^1$ | $R^2$ | $R^3$ | $R^4$ | $R^5$ | $R^6$ | $R^7$ | physical date |
|---|---|---|---|---|---|---|---|---|
| 1.154 | H | $Na^+$ | $CH_3$ | $CH_3$ | H | H | H | |
| 1.155 | H | $K^+$ | $CH_3$ | $CH_3$ | H | H | H | |
| 1.156 | H | $NH_4^+$ | $CH_3$ | $CH_3$ | H | H | H | |
| 1.157 | H | $N(CH_3)_4^+$ | $CH_3$ | $CH_3$ | H | H | H | |
| 1.158 | H | $N(CH_3)_4^+$ | H | H | H | H | H | |
| 1.159 | SH | $CH_3$ | benzyl | H | H | H | H | 1,2-trans |
| 1.160 | SH | $CH_3$ | benzyl | H | H | H | H | 1,2-cis |
| 1.161 | H | $CH_3$ | benzyl | H | H | H | H | 1,2-trans |
| 1.162 | H | $CH_3$ | benzyl | H | H | H | H | 1,2-cis |
| 1.163 | H | $CH_3$ | benzyl | H | H | H | H | 1,2-trans .$HNO_3$ |
| 1.164 | H | $CH_3$ | benzyl | H | H | H | H | 1,2-cis .$HNO_3$ |

Table 2:

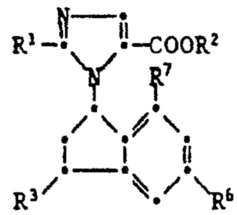

| Comp. No. | $R^1$ | $R^2$ | $R^3$ | $R^6$ | $R^7$ | physical data |
|---|---|---|---|---|---|---|
| 2.01 | H | $CH_3$ | H | H | F | |
| 2.02 | H | $CH_3$ | H | H | Cl | |
| 2.03 | H | $CH_3$ | H | H | Br | |
| 2.04 | H | $CH_3$ | $CH_3$ | Cl | Cl | |
| 2.05 | SH | $CH_3$ | H | H | F | |
| 2.06 | SH | $C_2H_5$ | H | H | F | |
| 2.07 | SH | $CH_3$ | H | H | Cl | |
| 2.08 | SH | $CH_3$ | H | Cl | Cl | |
| 2.09 | H | $C_2H_5$ | H | Cl | Cl | |
| 2.10 | SH | $CH_3$ | $CH_3$ | Cl | Cl | |
| 2.11 | H | $C_2H_5$ | $C_2H_5$ | H | H | |
| 2.12 | SH | $C_2H_5$ | $C_2H_5$ | H | H | |
| 2.13 | H | $C_3H_7$-n | $CH_3$ | H | H | |
| 2.14 | SH | $C_3H_7$-n | $C_3H_7$-n | H | H | |
| 2.15 | H | $C_3H_7$-n | $C_3H_7$-n | H | H | |
| 2.16 | SH | $CH_3$ | H | H | $OCH_3$ | |
| 2.17 | H | $CH_3$ | H | H | $OCH_3$ | |
| 2.18 | SH | $CH_3$ | H | $CH_3$ | H | |
| 2.19 | H | $CH_3$ | H | $CH_3$ | H | |
| 2.20 | H | $CH_3$ | H | H | H | $\cdot HNO_3$/m.p. 139,5-140,5°C |
| 2.21 | H | $CH_3$ | H | H | H | m.p.82-83°C |
| 2.22 | H | $C_2H_5$ | H | H | H | $\cdot HNO_3$/m.p.139-140°C |
| 2.23 | H | $C_2H_5$ | H | H | H | m.p.139-140°C |
| 2.24 | SH | $CH_3$ | H | F | H | m.p.191°C |
| 2.25 | H | $CH_3$ | H | F | H | m.p.187°C |
| 2.26 | SH | $CH_3$ | H | Cl | H | m.p.218°C |

Table 2: (continuation)

| Comp. No. | $R^1$ | $R^2$ | $R^3$ | $R^6$ | $R^7$ | physical data |
|---|---|---|---|---|---|---|
| 2.27 | H | $CH_3$ | H | Cl | H | m.p.214°C |
| 2.28 | H | $CH_3$ | H | Br | H | m.p.164°C |
| 2.29 | SH | $CH_3$ | $CH_3$ | Cl | Cl | m.p.203°C |
| 2.30 | H | $CH_3$ | $CH_3$ | Cl | Cl | m.p.126°C |
| 2.31 | SH | $CH_3$ | H | Br | H | |
| 2.32 | SH | $CH_3$ | H | H | H | mp.158-160.5°C |
| 2.33 | H | H | H | H | H | mp.209-211.5°C |
| 2.34 | SH | $CH_3$ | $CH_3$ | H | H | |
| 2.35 | H | $CH_3$ | $CH_3$ | H | H | |

Table 3

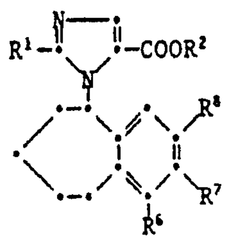

| Comp. No. | $R^1$ | $R^2$ | $R^6$ | $R^7$ | $R^8$ | physical data |
|---|---|---|---|---|---|---|
| 3.01 | SH | $CH_3$ | H | H | H | m.p.230°C(decomp.) |
| 3.02 | H | $CH_3$ | H | H | H | $\cdot HNO_3$/m.p.141-144°C |
| 3.03 | H | $C_6H_5$ | H | H | H | |
| 3.04 | H | $CH_3$ | Cl | H | Cl | |
| 3.05 | H | $CH_3$ | H | Cl | Cl | |
| 3.06 | SH | $C_2H_5$ | H | H | H | |
| 3.07 | SH | $CH_3$ | Cl | H | Cl | |
| 3.08 | SH | $CH_3$ | H | Cl | Cl | |
| 3.09 | H | $CH_3$ | H | H | F | |
| 3.10 | SH | $CH_3$ | H | H | F | |
| 3.11 | SH | $CH_3$ | $OCH_3$ | $CH_3$ | H | |
| 3.12 | H | $CH_3$ | $OCH_3$ | $CH_3$ | H | |
| 3.13 | SH | $CH_3$ | H | H | $CH_3$ | |
| 3.14 | H | $CH_3$ | H | H | $CH_3$ | |

Table 4

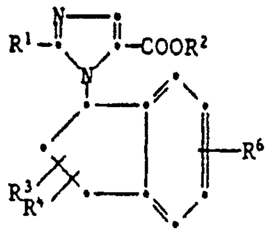

| Comp. No. | $R^1$ | $R^2$ | $R^3$ | $R^4$ | $R^6$ | phyical data |
|---|---|---|---|---|---|---|
| 4.01 | SH | $CH_3$ | $2-CH_3$ | $2-CH_3$ | H | m.p.171-172°C |
| 4.02 | H | $CH_3$ | $2-CH_3$ | $2-CH_3$ | H | $\cdot HNO_3$/m.p.172-173°C |
| 4.03 | H | $CH_3$ | $2-CH_3$ | $2-CH_3$ | H | m.p.96.5-97.5°C |
| 4.04 | SH | $CH_3$ | $2-C_2H_5$ | $2-C_2H_5$ | H | |
| 4.05 | H | $CH_3$ | $2-C_2H_5$ | $2-C_2H_5$ | H | $\cdot HNO_3$ |
| 4.06 | H | $CH_3$ | $2-C_2H_5$ | $2-C_2H_5$ | H | |
| 4.07 | SH | $CH_3$ | $2-C_3H_7-i$ | H | H | |
| 4.08 | H | $CH_3$ | $2-C_3H_7-i$ | H | H | |
| 4.09 | H | $CH_3$ | $2-C_3H_7-i$ | H | H | $\cdot HNO_3$ |
| 4.10 | SH | $CH_3$ | $2-CH_3$ | H | 5-Cl | |
| 4.11 | H | $CH_3$ | $2-CH_3$ | H | 5-Cl | |
| 4.12 | SH | $CH_3$ | $2-n-C_3H_7$ | H | H | |
| 4.13 | H | $CH_3$ | $2-n-C_3H_7$ | H | H | |
| 4.14 | SH | $CH_3$ | $2-n-C_4H_9$ | H | H | |
| 4.15 | H | $CH_3$ | $2-n-C_4H_9$ | H | H | |
| 4.16 | SH | $CH_3$ | 2-benzyl | H | H | |
| 4.17 | H | $CH_3$ | 2-benzyl | H | H | |
| 4.18 | SH | $CH_3$ | $n-C_5H_{11}$ | H | H | |
| 4.19 | H | $CH_3$ | $n-C_5H_{11}$ | H | H | |
| 4.20 | SH | $CH_3$ | $2-CH_3$ | H | H | |
| 4.21 | H | $CH_3$ | $2-CH_3$ | H | H | |
| 4.22 | SH | $CH_3$ | $2-C_2H_5$ | H | H | |
| 4.23 | H | $CH_3$ | $2-C_2H_5$ | H | H | |

Table 5:

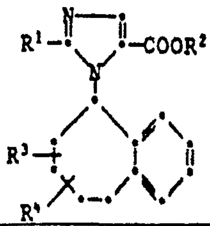

| Comp. No. | $R^1$ | $R^2$ | $R^3$ | $R^4$ |
|---|---|---|---|---|
| 5.01 | SH | $CH_3$ | H | H |
| 5.02 | H | $CH_3$ | H | H |
| 5.03 | H | benzyl | H | H |
| 5.04 | SH | benzyl | H | H |
| 5.05 | H | $CH_3$ | 2-$CH_3$ | 2-$CH_3$ |
| 5.06 | SH | $CH_3$ | 2-$CH_3$ | 2-$CH_3$ |
| 5.07 | H | $C_2H_5$ | 2-$CH_3$ | 2-$CH_3$ |
| 5.08 | S | $C_2H_5$ | 2-$CH_3$ | 2-$CH_3$ |

Table 6:

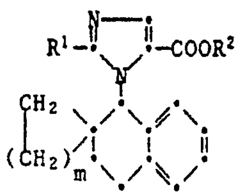

| Comp. No. | $R^1$ | $R^2$ | m | physical data |
|---|---|---|---|---|
| 6.01 | SH | $CH_3$ | 3 | m.p.161-164°C |
| 6.02 | H | $CH_3$ | 3 | •$HNO_3$/m.p.132°C (decomp.) |
| 6.03 | H | $CH_3$ | 3 | m.p.153-154°C |
| 6.04 | SH | $CH_3$ | 4 | |
| 6.05 | H | $CH_3$ | 4 | |
| 6.06 | H | $CH_3$ | 4 | •$HNO_3$ |
| 6.07 | H | $CH_2-CH=CH_2$ | 3 | |
| 6.08 | SH | $CH_2-CH=CH_2$ | 3 | |
| 6.09 | H | $CH_2CH_2-OCH_3$ | 4 | |
| 6.10 | SH | $CH_2-CH_2-OCH_3$ | 4 | |
| 6.11 | SH | $CH_3$ | 1 | |
| 6.12 | H | $CH_3$ | 1 | |
| 6.13 | SH | $CH_3$ | 2 | |
| 6.14 | H | $CH_3$ | 2 | |

**Table 7:**

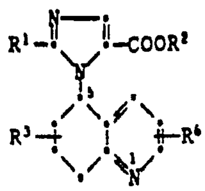

| Comp. No. | $R^1$ | $R^2$ | $R^3$ | $R^6$ | physical data |
|---|---|---|---|---|---|
| 7.01 | SH | $CH_3$ | H | 2-$CH_3$ | m.p.233° (decomp.) |
| 7.02 | H | $CH_3$ | H | 2-$CH_3$ | |
| 7.03 | H | $C_2H_5$ | 6-$CH_3$ | H | |
| 7.04 | SH | $C_2H_5$ | 6-$CH_3$ | H | |
| 7.05 | H | $CH_3$ | 6-$CH_3$ | H | |
| 7.06 | SH | $CH_3$ | 6-$CH_3$ | H | |

**Table 8:**

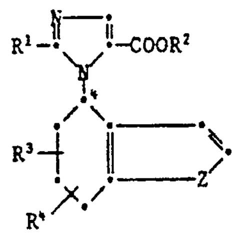

| Comp. No. | $R^1$ | $R^2$ | $R^3$ | $R^4$ | Z | physikal data |
|---|---|---|---|---|---|---|
| 8.01 | SH | $CH_3$ | 6-$CH_3$ | 6-$CH_3$ | O | m.p.180-182°C |
| 8.02 | SH | $CH_3$ | H | H | S | mp.166°C |
| 8.03 | H | $CH_3$ | H | H | S | •$HNO_3$/mp. 154°C |
| 8.04 | SH | $CH_3$ | 5-$CH_3$ | 5-$CH_3$ | S | |
| 8.05 | H | $CH_3$ | 5-$CH_3$ | 5-$CH_3$ | S | |
| 8.06 | H | $CH_3$ | 5-$CH_3$ | H | S | |
| 8.07 | SH | $CH_3$ | 5-$CH_3$ | H | S | |
| 8.08 | H | $CH_3$ | 5-$C_2H_5$ | H | S | |
| 8.09 | H | $CH_3$ | 5-$C_3H_7$-i | H | S | |
| 8.10 | H | $CH_3$ | 6-$CH_3$ | 6-$CH_3$ | O | |
| 8.11 | SH | $CH_3$ | H | H | O | |
| 8.12 | H | $CH_3$ | H | H | O | |
| 8.13 | SH | $C_2H_5$ | 5-$CH_3$ | 5-$CH_3$ | O | |
| 8.14 | H | $C_2H_5$ | 5-$CH_3$ | 5-$CH_3$ | O | |
| 8.15 | SH | $C_5H_9$-cycl. | 5-$CH_3$ | H | S | |
| 8.16 | H | $C_5H_9$-cycl. | 5-$CH_3$ | H | S | |

Table 9:

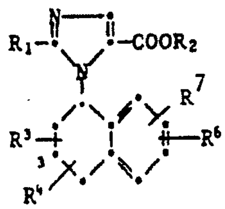

| Comp. No. | $R^1$ | $R^2$ | $R^3$ | $R^4$ | $R^6$ | $R^7$ | physical data |
|---|---|---|---|---|---|---|---|
| 9.01 | SH | $CH_3$ | 3-$CH_3$ | 3-$CH_3$ | H | H | |
| 9.02 | H | $CH_3$ | 3-$CH_3$ | 3-$CH_3$ | H | H | $\cdot HNO_3$ |
| 9.03 | H | $CH_3$ | 3-$CH_3$ | 3-$CH_3$ | H | H | |
| 9.04 | SH | $CH_3$ | 2-$CH_3$ | 3-$CH_3$ | H | H | |
| 9.05 | H | $CH_3$ | 2-$CH_3$ | 3-$CH_3$ | H | H | |
| 9.06 | H | $CH_3$ | 2-$CH_3$ | 3-$CH_3$ | H | H | $\cdot HNO_3$ |
| 9.07 | H | $CH_3$ | 2-$CH_3$ | 3-$CH_3$ | H | H | 2,3-cis |
| 9.08 | H | $CH_3$ | 2-$CH_3$ | 3-$CH_3$ | H | H | 2,3-trans |
| 9.09 | H | $C_2H_5$ | 3-$CH_3$ | H | 7-Br | H | |
| 9.10 | SH | $C_2H_5$ | 3-$CH_3$ | H | 7-Br | H | |
| 9.11 | H | $CH_3$ | 3-$CH_3$ | 3-$CH_3$ | 8-Cl | H | |
| 9.12 | SH | $CH_3$ | 3-$CH_3$ | 3-$CH_3$ | 8-Cl | H | |
| 9.13 | SH | $CH_3$ | H | H | 6-Cl | H | mp.181°C |
| 9.14 | H | $CH_3$ | H | H | 6-Cl | H | .$HNO_3$/mp.151°C |
| 9.15 | SH | $CH_3$ | H | H | 6-Cl | 7-Cl | mp.212°C |
| 9.16 | H | $CH_3$ | H | H | 6-Cl | 7-Cl | .$HNO_3$/mp.200°C |
| 9.17 | SH | $CH_3$ | 3-$CH_3$ | H | H | H | |
| 9.18 | H | $CH_3$ | 3-$CH_3$ | H | H | H | |
| 9.19 | SH | $CH_3$ | 3-$C_2H_5$ | H | H | H | |
| 9.20 | H | $CH_3$ | 3-$C_2H_5$ | H | H | H | |
| 9.21 | SH | $CH_3$ | 3-$C_2H_5$ | 3-$C_2H_5$ | H | H | |
| 9.22 | H | $CH_3$ | 3-$C_2H_5$ | 3-$C_2H_5$ | H | H | |

Table 10:

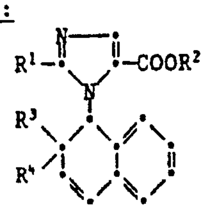

| Comp. No. | $R^1$ | $R^2$ | $R^3$ | $R^4$ |
|---|---|---|---|---|
| 10.01 | H | $CH_3$ | $CH_3$ | $CH_3$ |
| 10.02 | SH | $CH_3$ | $CH_3$ | $CH_3$ |
| 10.03 | SH | $CH_3$ | H | H |

Table 10: (continuation)

| Comp. No. | $R^1$ | $R^2$ | $R^3$ | $R^4$ |
|---|---|---|---|---|
| 10.04 | H | $CH_3$ | H | H |
| 10.05 | SH | $CH_3$ | $CH_3$ | H |
| 10.06 | H | $CH_3$ | $CH_3$ | H |
| 10.07 | SH | $C_4H_9$-n | H | H |
| 10.08 | H | $C_4H_9$-n | H | H |

Table 11:

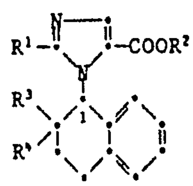

| Comp. No. | $R^1$ | $R^2$ | $R^3$ | $R^4$ | $[\alpha]_D$ | physical data |
|---|---|---|---|---|---|---|
| 11.01 | SH | $CH_3$ | H | H | +97.1° | m.p.141°C |
| 11.02 | SH | $CH_3$ | H | H | -87.9° | m.p.141°C |
| 11.03 | H | $CH_3$ | H | H | -25.8° | resin |
| 11.04 | H | $CH_3$ | H | H | +23.6° | resin |
| 11.05 | SH | $CH_3$ | $CH_3$ | $CH_3$ | | |
| 11.06 | SH | $CH_3$ | $CH_3$ | $CH_3$ | | |
| 11.07 | H | $CH_3$ | $CH_3$ | $CH_3$ | | |
| 11.08 | H | $CH_3$ | $CH_3$ | $CH_3$ | | |

Table 12:

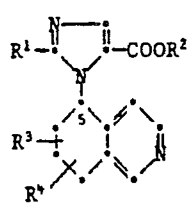

| Comp. No. | $R^1$ | $R^2$ | $R^3$ | $R^4$ |
|---|---|---|---|---|
| 12.01 | SH | $CH_3$ | H | H |
| 12.02 | H | $CH_3$ | H | H |
| 12.03 | SH | $CH_3$ | 6-$CH_3$ | H |
| 12.04 | H | $CH_3$ | 6-$CH_3$ | H |
| 12.05 | SH | $CH_3$ | 6-$CH_3$ | 6-$CH_3$ |
| 12.06 | H | $CH_3$ | 6-$CH_3$ | 6-$CH_3$ |
| 12.07 | SH | $CH_3$ | 6-$C_3H_7$-n | H |
| 12.08 | H | $CH_3$ | 6-$C_3H_7$-n | H |

**Table 13:**

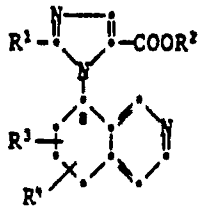

| Comp. No. | $R^1$ | $R^2$ | $R^3$ | $R^4$ |
|---|---|---|---|---|
| 13.01 | SH | $CH_3$ | H | H |
| 13.02 | H | $CH_3$ | H | H |
| 13.03 | SH | $CH_3$ | 7-$CH_3$ | H |
| 13.04 | H | $CH_3$ | 7-$CH_3$ | H |
| 13.05 | SH | $CH_3$ | 7-$CH_3$ | 7-$CH_3$ |
| 13.06 | H | $CH_3$ | 7-$CH_3$ | 7-$CH_3$ |
| 13.07 | H | $CH_3$ | 7-benzyl | H |
| 13.08 | H | $CH_3$ | 7-benzyl | H |
| 13.09 | H | $C_2H_5$ | 7-allyl | H |
| 13.10 | H | $C_2H_5$ | 7-allyl | H |

**Table 14:**

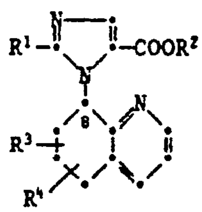

| Comp. No. | $R^1$ | $R^2$ | $R^3$ | $R^4$ |
|---|---|---|---|---|
| 14.01 | SH | $CH_3$ | H | H |
| 14.02 | H | $CH_3$ | H | H |
| 14.03 | SH | $CH_3$ | 7-$CH_3$ | H |
| 14.04 | H | $CH_3$ | 7-$CH_3$ | H |
| 14.05 | SH | $CH_3$ | 7-$CH_3$ | 7-$CH_3$ |
| 14.06 | H | $CH_3$ | 7-$CH_3$ | 7-$CH_3$ |
| 14.07 | SH | $C_3H_7$-i | H | H |
| 14.08 | H | $C_3H_7$-i | H | H |

**Table 15:**

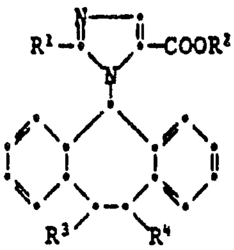

| Comp. No. | $R^1$ | $R^2$ | $R^3$ | $R^4$ |
|---|---|---|---|---|
| 15.01 | SH | $CH_3$ | H | H |
| 15.02 | H | $CH_3$ | H | H |
| 15.03 | H | $C_2H_5$ | H | H |
| 15.04 | H | $C_2H_5$ | $CH_3$ | H |
| 15.05 | H | $CH_3$ | $CH_3$ | $CH_3$ |
| 15.06 | H | $C_5H_{11}$-n | H | H |
| 15.07 | H | $C_6H_{11}$-cycl. | H | H |
| 15.08 | H | $C_3H_7$-i | H | H |

**Table 16:**

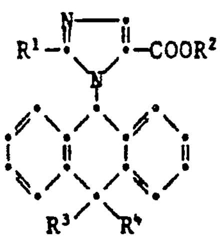

| Comp. No. | $R^1$ | $R^2$ | $R^3$ | $R^4$ |
|---|---|---|---|---|
| 16.01 | SH | $CH_3$ | $CH_3$ | $CH_3$ |
| 16.02 | H | $CH_3$ | $CH_3$ | $CH_3$ |
| 16.03 | H | $C_2H_5$ | $CH_3$ | $CH_3$ |
| 16.04 | H | $CH_2$-$CH_2$-$OCH_3$ | $CH_3$ | $CH_3$ |
| 16.05 | H | $C_5H_9$-cycl. | $CH_3$ | $CH_3$ |
| 16.06 | H | $C_4H_9$-n | $CH_3$ | $CH_3$ |
| 16.07 | SH | $CH_3$ | $C_2H_5$ | $C_2H_5$ |
| 16.08 | H | $CH_3$ | $C_2H_5$ | $C_2H_5$ |
| 16.09 | SH | $CH_3$ | $-(CH_2)_5-$ | |
| 16.10 | H | $CH_3$ | $-(CH_2)_5-$ | |

**Table 17:**

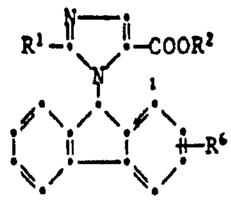

| Comp. No. | $R^1$ | $R^2$ | $R^6$ | physical data |
|---|---|---|---|---|
| 17.01 | H | $CH_3$ | H | mp.146°C |
| 17.02 | SH | $CH_3$ | H | |
| 17.03 | H | $CH_3$ | 1-$CH_3$ | |
| 17.04 | SH | $CH_3$ | 1-$CH_3$ | |
| 17.05 | H | $C_2H_5$ | H | |
| 17.06 | H | $C_2H_5$ | 1-$CH_3$ | |
| 17.07 | H | H | H | mp.>260°C(dec.) |
| 17.08 | SH | H | H | |
| 17.09 | H | H | 1-$CH_3$ | |
| 17.10 | SH | H | 1-$CH_3$ | |

Table 18:

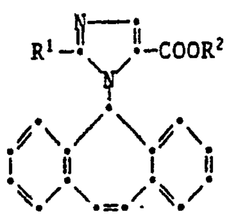

| Comp. No. | $R^1$ | $R^2$ |
|---|---|---|
| 18.01 | SH | $CH_3$ |
| 18.02 | H | $CH_3$ |
| 18.03 | H | $C_2H_5$ |
| 18.04 | H | $C_6H_{11}$-cycl. |

Table 19:

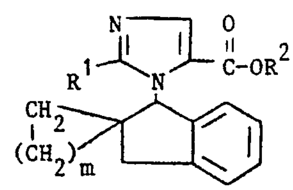

| Comp. No. | $R^1$ | $R^2$ | m |
|---|---|---|---|
| 19.01 | SH | $CH_3$ | 1 |
| 19.02 | H | $CH_3$ | 1 |
| 19.03 | SH | $CH_3$ | 2 |
| 19.04 | H | $CH_3$ | 2 |
| 19.05 | SH | $CH_3$ | 3 |
| 19.06 | H | $CH_3$ | 3 |
| 19.07 | SH | $CH_3$ | 4 |
| 19.08 | H | $CH_3$ | 4 |

The following examples are intended to illustrate the present invention in all its aspects and not to limit it thereto.

A. Composition Examples

Example 1: Formulation Examples for solid compounds of formula I (percentages are by weight)

| a) Wettable powders | a) | b) | c) |
|---|---|---|---|
| compound No. 1.02 | 20 % | 50 % | 0.5 % |
| sodium lignosulfonate | 5 % | 5 % | 5 % |
| sodium laurylsulfate | 3 % | - | - |
| sodium diisobutylnaphthalenesulfonate | - | 6 % | 6 % |
| octylphenol polyethylene glycol ether (7-8 moles of ethylene oxide) | - | 2 % | 2 % |
| highly dispersed silicic acid | 5 % | 27 % | 27 % |
| kaolin | 67 % | - | - |
| sodium chloride | - | - | 59.5 % |

The active ingredient is thoroughly mixed with the adjuvants and the mixture is thoroughly ground in a suitable mill, affording wettable powders which can be diluted with water to give suspensions of the desired concentration.

| b) Emulsifiable concentrate | a) | b) |
|---|---|---|
| compound No. 1.03 | 10 % | 1 % |
| octylphenol polyethylene glycol ether (4-5 moles of ethylene oxide) | 3 % | 3 % |
| calcium dodecylbenzenesulfonate | 3 % | 3 % |
| castor oil polyglycol ether (36 moles of ethylene oxide) | 4 % | 4 % |
| cyclohexanone | 30 % | 10 % |
| xylene mixture | 50 % | 79 % |

Emulsions of any required concentration can be obtained from this concentrate by dilution with water.

| c) Dusts | a) | b) |
|---|---|---|
| compound No. 1.03 | 0.1 % | 1 % |
| talcum | 99.9 % | - |
| kaolin | - | 99 % |

Dusts which are ready for use are obtained by mixing the active ingredient with the carriers, and grinding the mixture in a suitable mill.

d) Extruder granulate

| | a) | b) |
|---|---|---|
| compound No. 1.03 | 10 % | 1 % |
| sodium lignosulfonate | 2 % | 2 % |
| carboxymethylcellulose | 1 % | 1 % |
| kaolin | 87 % | 96 % |

The active ingredient is mixed and ground with the adjuvants, and the mixture is subsequently moistened with water. The mixture is extruded and then dried in a stream of air.

e) Coated granulate

| | |
|---|---|
| compound No. 1.03 | 3 % |
| polyethylene glycol (mol.wt. 200) | 2 % |
| kaolin | 94 % |

The finely ground active ingredient is uniformly applied, in a mixer, to the kaolin moistened with polyethylene glycol. Non-dusty coated granulates are obtained in this manner.

f) Suspension concentrate

| | a) | b) |
|---|---|---|
| compound No. 1.02 | 40 % | 5 % |
| ethylene glycol | 10 % | 10 % |
| nonylphenol polyethylene glycol ether (15 moles of ethylene oxide) | 6 % | 1 % |
| sodium lignosulfonate | 10 % | 5 % |
| carboxymethylcellulose | 1 % | 1 % |
| 37 % aqueous formaldehyde solution | 0.2 % | 0.2 % |
| silicone oil in the form of a 75 % aqueous emulsion | 0.8 % | 0.8 % |
| water | 32 % | 77 % |

The finely ground active ingredient is intimately mixed with the adjuvants, giving a suspension concentrate from which suspensions of any desired concentration can be obtained by dilution with water.

g) Salt solution

| | |
|---|---|
| compound No. 1.02 | 5 % |
| isopropylamine | 1 % |
| octylphenol polyethylene glycol ether (78 moles of ethylene oxide) | 3 % |
| water | 91 % |

Example 2:

Formulation Examples for liquid active ingredients of formula I (throughout, percentages are by weight)

| a) Emulsifiable concentrates | a) | b) | c) |
|---|---|---|---|
| compound No. 1.60 | 20 % | 40 % | 50 % |
| calcium dodecylbenzenesulfonate | 5 % | 8 % | 5.8 % |
| castor oil polyethylene glycol ether (36 moles of ethylene oxide) | 5 % | - | - |
| tributylphenol polyethylene glycol ether (30 moles of ethylene oxide) | - | 12 % | 4.2 % |
| cyclohexanone | - | 15 % | 20 % |
| xylene mixture | 70 % | 25 % | 20 % |

Emulsions of any required concentration can be produced from such concentrates by dilution with water.

| b) Solutions | a) | b) | c) | d) |
|---|---|---|---|---|
| compound No. 1.60 | 80 % | 10 % | 5 % | 95 % |
| ethylene glycol monomethyl ether | 20 % | - | - | - |
| polyethylene glycol (MG 400) | - | 70 % | - | - |
| N-methyl-2-pyrrolidone | - | 20 % | - | - |
| epoxidised coconut oil | - | - | 1 % | 5 % |
| petroleum distillate (boiling range 160-190°) | - | - | 94 % | - |

These solutions are suitable for application in the form of microdrops.

| c) Granulates | a) | b) |
|---|---|---|
| compound No. 1.60 | 5 % | 10 % |
| kaolin | 94 % | - |
| highly dispersed silicic acid | 1 % | - |
| attapulgite | - | 90 % |

The active ingredient is dissolved in methylene chloride, the solution is sprayed onto the carrier, and the solvent is subsequently evaporated off in vacuo.

| d) Dusts | a) | b) |
|---|---|---|
| a compound No. 1.60 | 2 % | 5 % |
| highly dispersed silicic acid | 1 % | 5 % |
| talcum | 97 % | - |
| kaolin | - | 90 % |

Ready-for-use dusts are obtained by intimately mixing the carriers with the active ingredient.

## B. Biological Examples

### Example 3 Preemergence herbicidal action

In a greenhouse, immediately after sowing the test plants in seed dishes, the surface of the soil is treated with an aqueous dispersion of the test compounds, obtained from a 25 % emulsifiable concentrate or from a 25 % wettable powder with test compounds, which, on account of their insufficient solubility, cannot be formulated to emulsifiable concentrates. Two different concentration series were used, corresponding to 1 and 0.5 kg of test compound per hectare respectively. The seed dishes are kept in the greenhouse at 22°-25° C and 50-70 % relative humidity. The test is evaluated 3 weeks later in accordance with the following rating:

1 = plants have not germinated or are totally withered
2-3 = very strong action
4-6 = average action
7-8 = slight action
9 = no action

In this test, the tested compounds of formula(I) were most effective against monocot grass weeds, whereas no or only insignificant damage was caused to cultivated plants such as maize at the given rates of application.

Results: Preemergence test

| dosage kg a.i./ha / plant tested | Comp. 1.01 | | Comp. 1.02 | | Comp. 1.03 | | Comp. 1.08 | |
|---|---|---|---|---|---|---|---|---|
| | 1 | 0.5 | 1 | 0.5 | 1 | 0.5 | 1 | 0.5 |
| maize | 9 | 9 | 8 | 9 | 8 | 9 | 9 | 9 |
| alopecurus myos. | 2 | 2 | 2 | 2 | 2 | 2 | 2 | 2 |
| digitaria sang. | - | - | 1 | 1 | 1 | 1 | 1 | 1 |
| echinochloa c.g. | 1 | 1 | 1 | 3 | 1 | 1 | 1 | 1 |
| sida spinosa | - | - | 4 | 4 | 1 | 1 | 2 | 3 |
| amaranthus ret. | - | - | 4 | 4 | 1 | 1 | 2 | 2 |
| chenopodium sp. | 3 | 3 | 3 | 3 | 1 | 1 | 1 | 1 |
| solanum nigrum | - | - | 3 | 3 | 1 | 1 | 1 | 1 |
| chrysanthe.leuc. | - | - | 2 | 2 | 2 | 2 | 2 | 2 |
| galium aparine | 3 | 4 | 2 | 2 | 1 | 1 | 2 | 3 |
| viola tricolor | 3 | 3 | 2 | 3 | 1 | 1 | 1 | 1 |
| veronica sp. | - | - | 2 | 2 | 1 | 1 | 1 | 1 |

-: not tested

Results: Preemergence test

| dosage kg a.i./ha / plant tested | Comp. 1.16 | | Comp. 1.17 | | Comp. 4.01 | |
|---|---|---|---|---|---|---|
| | 1 | 0.5 | 1 | 0.5 | 1 | 0.5 |
| maize | 9 | 9 | 9 | 9 | 8 | 9 |
| alopecurus myos. | 5 | 6 | 2 | 2 | 3 | 4 |
| digitaria sang. | 1 | 1 | 1 | 1 | 1 | 1 |
| echinochloa c.g. | 2 | 2 | 1 | 1 | 1 | 1 |
| sida spinosa | 3 | 3 | 1 | 2 | 2 | 3 |
| amaranthus ret. | 3 | 3 | 2 | 2 | 2 | 3 |
| chenopodium sp. | 3 | 4 | 1 | 1 | 1 | 1 |
| solanum nigrum | 2 | 2 | 1 | 1 | 2 | 2 |
| chrysanthe.leuc. | 3 | 3 | 2 | 2 | 4 | 6 |
| galium aparine | 4 | 5 | 2 | 3 | 2 | 3 |
| viola tricolor | 3 | 4 | 1 | 1 | 2 | 2 |
| veronica sp. | 2 | 2 | 1 | 1 | 1 | 1 |

Example 4 : Postemergence herbicidal action (Contact herbicide):

A large number of weeds and cultivated plants are sprayed post-emergence in the 4- to 6-leaf stage with an aqueous active ingredient dispersion in rates of 4 and 2 kg of test compound per hectare and kept at 24°-26°C and 45-60 % relative humidity. The test was evaluated at least 15 days after treatment in accordance with the same rating as employed in the preemergence test.

In this test, the compounds of formula I were also most effective against the tested weeds. The cultivated plants such as maize and rice were either not damaged or only damaged at higher application rates of the tested compound.

Results: Postemergence test

| dosage g.a.i./ha | Comp. 1.06 | |
|---|---|---|
| plant tested | 4 | 2 |
| maize | 8 | 9 |
| rice, dry | 9 | 9 |
| xanthium sp. | 3 | 4 |
| chenopodium sp. | 4 | 5 |
| ipomosa | 4 | 5 |
| sinapis | 4 | 7 |
| galium aparine | 5 | 5 |
| viola tricolor | 4 | 6 |

Example 5 : Herbicidal action in transplanted rice crops

25 days old rice shoots of the variety "Yamabiko" were transplanted into large plastic containers. Into the same containers seeds of the weeds occurring in rice crops, namely alisma, ammonia, cyperus, echinochloa, eleocharis, fimbristylis, scirpus and monochoria, were sown between the rice plants. The containers were watered to such an extent, that a water layer of 2.5 cm covers the surface. After 3 days under green house conditions, the diluted aqueous dispersions of the active compounds were added to the water layer at a rate of application of 500, 250, 125, 60, 30 and 15 g a.i. per hectare. The containers were then kept covered with water at a temperature of 25° C and high humidity in a greenhouse for 4 weeks. The evaluation of the tests was made in accordance with the rating given in Example 3.

Results: (-: not tested)

| Compound No. / Tested plant | 1.01 in g a.i. per hectare 500 | 250 | 125 | 60 | 30 | 15 | 1.02 in g a.i. per hectare 500 | 250 | 125 | 60 | 30 | 15 |
|---|---|---|---|---|---|---|---|---|---|---|---|---|
| rice "Yamabiko" | 8 | 9 | 9 | 9 | 9 | 9 | 8 | 9 | 9 | 9 | 9 | 9 |
| alisma | 1 | 1 | 1 | 1 | 2 | 3 | 1 | 2 | 2 | 3 | 3 | 3 |
| ammania | 1 | 2 | 2 | 2 | 2 | 2 | - | - | - | - | - | - |
| cyperus difformia | 1 | 1 | 1 | 1 | 1 | 1 | 1 | 1 | 1 | 1 | 1 | 1 |
| echinochloa c.g. | 1 | 1 | 1 | 1 | 6 | 8 | 1 | 1 | 1 | 1 | 3 | 6 |
| eleocharis | - | - | - | - | - | - | 1 | 1 | 1 | 1 | 1 | 1 |
| fimbristylis | 1 | 1 | 1 | 1 | 1 | 1 | 1 | 1 | 1 | 1 | 1 | 1 |
| scirpus | 1 | 1 | 1 | 1 | 2 | 4 | 1 | 1 | 1 | 3 | 4 | 4 |
| monochoria | 1 | 1 | 1 | 1 | 1 | 3 | 1 | 1 | 1 | 1 | 1 | 1 |

| Compound No. / Tested plant | 1.03 in g a.i. per hectare 500 | 250 | 125 | 60 | 30 | 15 | 1.04 in g a.i. per hectare 500 | 250 | 125 | 60 | 30 | 15 |
|---|---|---|---|---|---|---|---|---|---|---|---|---|
| rice "Yamabiko" | 9 | 9 | 9 | 9 | 9 | 9 | 8 | 9 | 9 | 9 | 9 | 9 |
| alisma | 1 | 1 | 3 | 3 | 3 | 6 | 1 | 1 | 2 | 3 | 5 | 7 |
| cyperus difformia | 1 | 1 | 1 | 1 | 2 | 2 | 1 | 1 | 1 | 1 | 3 | 3 |
| echinochloa c.g. | 1 | 1 | 3 | 6 | 9 | 9 | 2 | 5 | 8 | 8 | 9 | 9 |
| eleocharis | 1 | 1 | 3 | 5 | 5 | 5 | 3 | 5 | 6 | 7 | 8 | 8 |
| fimbristylis | 1 | 1 | 1 | 1 | 1 | 1 | 1 | 1 | 1 | 1 | 1 | 1 |
| scirpus | 1 | 1 | 3 | 5 | 7 | 9 | 1 | 7 | 7 | 7 | 9 | 9 |
| monochoria | 1 | 1 | 1 | 1 | 3 | 9 | 1 | 1 | 3 | 5 | 5 | 6 |

| Compound No. / Tested plant | 1.11 in g a.i. per hectare 500 | 250 | 125 | 60 | 30 | 15 | 1.12 in g a.i. per hectare 500 | 250 | 125 | 60 | 30 | 15 |
|---|---|---|---|---|---|---|---|---|---|---|---|---|
| rice "Yamabiko" | 8 | 9 | 9 | 9 | 9 | 9 | 9 | 9 | 9 | 9 | 9 | 9 |
| alisma | 1 | 1 | 3 | 3 | 3 | 6 | 1 | 1 | 1 | 2 | 6 | 7 |
| ammania | - | - | - | - | - | - | 1 | 2 | 2 | 2 | 2 | 3 |
| cyperus difformis | 1 | 1 | 1 | 1 | 1 | 1 | 1 | 1 | 1 | 1 | 1 | 1 |
| echinochloa c.g. | 1 | 2 | 6 | 8 | 9 | 9 | 1 | 3 | 4 | 8 | 9 | 9 |
| eleocharis | 1 | 1 | 1 | 1 | 1 | 1 | - | - | - | - | - | - |
| fimbristylis | 1 | 1 | 1 | 1 | 1 | 1 | 1 | 1 | 1 | 1 | 1 | 2 |
| scirpus | 1 | 1 | 5 | 6 | 7 | 8 | 1 | 1 | 3 | 7 | 9 | 9 |
| monochoria | 1 | 1 | 1 | 2 | 4 | 6 | 1 | 1 | 2 | 2 | 4 | 7 |

| Compound No. / Tested plant | 1.16 in g a.i. per hectare 500 | 250 | 125 | 60 | 2.01 in g a.i. per hectare 500 | 250 | 125 | 60 |
|---|---|---|---|---|---|---|---|---|
| rice "Yamabiko" | 8 | 9 | 9 | 9 | 9 | 9 | 9 | 9 |
| echinochloa c.g. | 1 | 1 | 2 | 3 | 1 | 2 | 5 | 6 |
| scirpus | 1 | 1 | 1 | 2 | 1 | 1 | 3 | 4 |
| monochoria | 2 | 3 | 3 | 3 | 1 | 2 | 4 | 5 |

| Compound No. / Tested plant | 3.01 in g a.i. per hectare 500 | 250 | 125 | 4.01 in g a.i. per hectare 250 | 125 | 60 |
|---|---|---|---|---|---|---|
| rice "Yamabiko" | 9 | 9 | 9 | 9 | 9 | 9 |
| echinochloa c.g. | 3 | 4 | 5 | 1 | 1 | 1 |
| scirpus | 1 | 2 | 3 | 1 | 2 | 3 |
| monochoria | 2 | 2 | 4 | 2 | 2 | 3 |

## C. Preparatory Examples

Example 6: 1-(7-Methoxy-1,2,3,4-tetrahydronaphthalen-1-yl)-2-mercapto-5-imidazolecarboxylic acid methyl ester (compound 1.13).

a) N-(7-Methoxy-1,2,3,4-tetrahydronaphthalen-1-yl)glycine methyl ester.
367.0 g of 1-amino-7-methoxy-1,2,3,4-tetrahydronaphthalene are solved in 1500 ml of diethyl ether. To this solution is dropwise added a solution of 158.3 g of bromoacetic acid methyl ester in 450 ml of diethyl ether. The mixture is stirred at room temperature for 70 hours. The precipitate is separated and the solution is concentrated to dryness, yielding N-(7-methoxy-1,2,3,4-tetrahydronaphthalen-1-yl) glycine methyl ester quantitatively.
b) N-Formyl-N-(7-methoxy-1,2,3,4-tetrahydronaphthalen-1-yl) glycine methyl ester.
2.40 g of N-(7-methoxy-1,2,3,4-tetrahydronaphthalen-1-yl)glycine methyl ester are added dropwise with cooling to 5 °C to 5.52 g of formic acid. Additionally 1.79 g of acetic anhydride are added, and the mixture is kept at room temperature for 70 hours. Destillation under vacuum affords 2.7 g (97 % of theory) of N-formyl-N-(7-methoxy-1,2,3,4-tetrahydronaphthalen-1-yl)glycine methyl ester as a colourless resin.
c) A solution of 1.85 g of sodium methylate in 38 ml of tetrahydrofuran is prepared by adding suitable amounts of methanol and sodium hydride to the tetrahydrofuran. With optional cooling to room

temperature 6.5 g of formic acid methyl ester and 10.0 g of N-formyl-N-(7-methoxy-1,2,3,4-tetrahydronaphthalen-1-yl)glycine methyl ester are added. After 20 hours the mixture is taken up with 30 ml of deionisized water and 50 ml of diethyl ether. The aqueous phase is separated, and 30 ml of methanol and 8.6 ml of 36 % hydrochloric acid are added. The solution is heated to 40°-45° C and treated with a solution of 5.8 g of potassium thiocyanate in 10 ml of deionisized water. After 24 hours the mixture is heated to 80° C for 5 hours. Upon cooling 7.3 g (64 % of theory) of 1-(7-methoxy-1,2,3,4-tetrahydronaphthalen-1-yl)-2-mercapto-5-imidazolecarboxylic acid methyl ester precipitate, having a melting point of 184-186° C.

Example 7: 1-(7-Methoxy-1,2,3,4-tetrahydronaphthalen-1-yl)-5-imidazolecarboxylic acid methyl ester - (compound 1.05).

0.22 g of sodium nitrite and 2.85 g of nitric acid are solved in 7 ml of deionisized water. Within 1 hour 3.2 g of 1-(7-methoxy-1,2, 3,4-tetrahydronaphthalen-1-yl)-2-mercapto-5-imidazolecarboxylic acid methyl ester are added portionwise at a temperature between 25° C and 30° C. The precipitate is isolated affording 2.72 g of the nitric acid addition salt of 1-(7-methoxy-1,2,3,4-tetrahydronaphthalen-1-yl)-5-imidazolecarboxylic acid methyl ester. This salt is treated with 10 ml of 10 % aqueous sodium carbonate solution. Extracting the aqueous phase with chloroform, and evaporating the organic solvent yields 2.72 g of 1-(7-methoxy-1,2,3,4-tetrahydronaphthalen-1-yl)-5-imidazolecarboxylic acid methyl ester as a colourless resin.

Example 8 : 1-(1,2,3,4-Tetrahydronaphthalen-1-yl)-5-imidazolecarboxylic acid methyl ester (compound 1.02).

33.0 g of ammonium carbonate are added at room temperature to a solution of 15.5 g of N,α-bis-formyl-N-(1,2,3,4-tetrahydronaphthalen-1-yl)glycine methyl ester in 300 ml of xylene. The mixture is heated to 70° C for 1 hour then the temperature is raised to 120° C for 3 hours. After concentration the 1-(1,2,3,4-tetrahydronaphthalen-1-yl)-5-imidazolecarboxylic acid methyl ester precipitates from the solution, having a melting point of 63° C.

Example 9: 1-(1,2,3,4-Tetrahydronaphthalen-1-yl)-5-imidazolecarboxylic acid methyl enter (compound 1.02).

A mixture of 16.5 g of N,α-bis-formyl-N-(1,2,3,4-tetrahydronaphthalen-1-yl)glycine methyl ester, 65.0 g of ammonium acetate and 100 ml of acetic acid are refluxed for 8 hours. Then additional 50 g ammonium acetate are added and the refluxing is continued for further 4 hours. The solution is diluted with 300 ml of water and extracted twice, with 100 ml of toluene each time. The organic phases are combined, concentrated and separated at silica gel by chromatography. Concentration of the eluate yields 1-(1,2,3,4-tetrahydronaphthalen-1-yl)-5-imidazolecarboxylic acid methyl ester, having a melting point of 63° C.

Example 10: 1-(1,2,3,4-Tetrahydronaphthalen-1-yl)-5-imidazolecarboxylic acid methyl ester (compound 1.02).

A mixture of 16.5 g of N,α-bis-formyl-N-(1,2,3,4-tetrahydronaphthalen-1-yl)glycine methyl ester, 50 ml of formamide and 10 ml of hydrochloric acid are heated to 140° C for 8 hours. After cooling to room temperature the mixture is extracted with a mixture of 100 ml of water and 100 ml of diethyl ether. The ethereal phase is separated and the aqueous phase is extracted twice, with 100 ml of diethyl ether each time. The combined organic phases are dried over sodium sulfate and concentrated to dryness. The residue crystallizes affording pure 1-(1,2,3,4-tetrahdronaphthalen-1-yl)-5-imidazolecarboxylicacid methyl ester with a melting point of 63° C.

Example 11: 1-(2,2-Dimethyl-1,2,3,4-tetrahydronaphthalen-1-yl)-2-mercapto-5-imidazolecarboxylic acid methyl ester (compound 1.17).

a) N-(2,2-Dimethyl-1,2,3,4-tetrahydronaphthalen-1-yl)glycine methyl ester.
65.2 g of 1-amino-2,2-dimethyl-1,2,3,4-tetrahydronaphthalene are solved in 250 ml of diethyl ether. To this solution is dropwise added a solution of 28.5 g of bromoacetic acid methyl ester in 150 ml of diethyl ether. The mixture is stirred at room temperature for 70 hours. The precipitate is separated and the solution is concentrated to dryness, yielding N-(2,2-dimethyl-1,2,3,4-tetrahydronaphthalen-1-yl) glycine methyl ester quantitatively.
b) N-Formyl-N-(2,2-dimethyl-1,2,3,4-tetrahydronaphthalen-1-yl)-glycine methyl ester.

The complete yield of N-(2,2-dimethyl-1,2,3,4-tetrahydronaphthalen-1-yl)glycine methyl ester of the process a) is added dropwise with cooling to 5° C to 67.8 ml of formic acid. Additionally 24.9 ml of acetic anhydride are added, and the mixture is kept at room temperature for 70 hours. Destillation under vacuum affords 35.2 g of N-formyl-N-(2,2-dimethyl-1,2,3,4-tetrahydronaphthalen-1-yl)-glycine methyl ester having a melting point of 74-75° C.

c) A solution of 24.6 g of sodium methylate in 500 ml of tetrahydrofuran is prepared by adding suitable amounts of methanol and sodium hydride to the tetrahydrofuran. With optional cooling to room temperature 73.7 g of formic acid methyl ester and 35.2 g of N-formyl-N-(2,2-dimethyl-1,2,3,4-tetrahydronaphthalen-1-yl)glycine methyl ester are added. After 20 hours the mixture is taken up with 350 ml of deionisized water and 700 ml of diethyl ether. The aqueous phase is separated, and 350 ml of methanol and 97.7 ml of 36 % hydrochloric acid are added. The solution is heated to 40°-45° C and treated with a solution of 66.1 g of potassium thiocyanate in 200 ml of deionisized water. After 24 hours at 40° C the mixture is heated to 80° C for 5 hours. Upon cooling 35.8 g of 1-(2,2-dimethyl-1,2,3,4-tetrahydronaphthalen-1-yl)-2-mercapto-5-imidazole-carboxylic acid methyl ester precipitate, having a melting point of 171-172° C.

Example 12 : 1-(2,2-Dimethyl-1,2,3,4-tetrahydronaphthalen-1-yl)-5-imidazolecarboxylic acid methyl ester - (compound 1.03).

5.8 g of sodium nitrite and 55 g of nitric acid are solved in 3100 ml of deionisized water. Within 1 hour 92.2 g of 1-(2,2-dimethyl-1,2,3,4-tetrahydronaphthalen-1-yl)-2-mercapto-5-imidazolecarboxylic acid methyl ester are added portionswise at a temperature between 25° C and 30° C. The precipitate is isolated affording 100.0 g of the nitric acid addition salt of 1-(2,2-dimethyl-1,2,3,4-tetrahydronaphthalen-1-yl)-5-imidazolecarboxylic acid methyl ester. This salt is treated with 250 ml of 10 % aqueous sodium carbonate solution. Extracting the aqueous phase with chloroform, and evaporating the organic solvent yields 80.4 g of 1-(2,2-dimethyl-1,2,3,4-tetrahydronaphthalen-1-yl)-5-imidazolecarboxylic acid methyl ester having a melting point of 100-101° C.

Example 13: R-1-(1,2,3,4-Tetrahydronaphthalen-1-yl)-2-mercapto-5-imidazolecarboxylic acid methyl ester - (compound 11.01).

a) R-N-(1,2,3,4-Tetrahydronaphthalen-1-yl)-glycine methyl ester.

The optically pure isomer of R-1-amino-1,2,3,4-tetrahydronaphthalene was prepared as described in US Patent No. 3,953,506

51.4 g of R-1-amino-1,2,3,4-tetrahydronaphthalene and 37.1 g of sodium carbonate are dispersed in 300 ml of methanol. 53.6 g of bromoacetic acid methyl ester are added dropwise to this dispersion. The mixture is stirred at room temperature for 100 hours. The precipitate is separated and the solution is concentrated to dryness, yielding the R-N-(1,2,3,4-tetrahydronaphthalen-1-yl) glycine methyl ester quantitatively.

b) R-N-Formyl-N-(1,2,3,4-tetrahydronaphthalen-1-yl)glycine methyl ester.

64.9 g of R-N-(1,2,3,4-tetrahydronaphthalen-1-yl)glycine methyl ester are added dropwise with cooling to 5° C to 134 ml of formic acid. Additionally 48.9 ml of acetic anhydride are added, and the mixture is kept at room temperature for 70 hours. Destillation under vacuum affords 50.6 g of R-N-formyl-N-(1,2,3,4-tetrahydronaphthalen-1-yl)glycine methyl ester having a melting point of 83-84° C.

c) A solution of 11.2 g of sodium methylate in 250 ml of tetrahydrofuran is prepared by adding suitable amounts of methanol and sodium hydride to the tetrahydrofuran. With optional cooling to room temperature 35.1 g of formic acid methyl ester and the R-N-formyl-N-(1,2,3,4-tetrahydronaphthalen-1-yl)-glycine methyl eater obtained from the process b) are added. After 20 hours the mixture is taken up with 130 ml of deionisized water and 250 ml of diethyl ether. The aqueous phase is separated, aid 130 ml of methanol and 46.9 ml of 36 % hydrochloric acid are added. The solution is heated to 40°-50° C and treated with a solution of 31.5 g of potassium thiocyanate in 60 ml of deionisized water. The mixture is stirred at room temperature for 24 hours. During this period 24.4 g of R-1-(1,2,3,4-tetrahydronaphthalen-1-yl)-2-mercapto-5-imidazolecarboxylic acid methyl ester precipitate. After recrystallisation from methanol the product has a melting point of 141-142° C, $[\alpha]_D^{20} = +97.1°$.

Example 14: R-1-(1,2,3,4-Tetrahydronaphthalen-1-yl)-5-imidazolecarboxylic acid methyl ester (compound 11.03).

1.4 g of sodium nitrite and 11.7 ml of nitric acid are solved in 75 ml of deionisized water. Within 1 hour 17.9 g of R-1-(1,2,3,4-tetrahydronaphthalen-1-yl)-2-mercapto-5-imidazolecarboxylic acid methyl ester are added portionwise at a temperature between 25° C and 30° C. The precipitate is isolated affording the nitric acid addition salt of R-1-(1,2,3,4-tetrahydronaphthalen-1-yl)-5-imidazolecarboxylic acid methyl ester. This salt is treated with 10 % aqueous sodium carbonate solution. Extracting the aqueous phase with chloroform, and evaporating the organic solvent yields 7.4 g of R-1-(1,2,3,4-tetrahydronaphthalen-1-yl)-5-imidazolecarboxylic acid methyl ester as a colourless resin, which is purified by chromatography through a silica column. The pure resinous product has a refraction index $[\alpha]_D^{20}$ = -25.80°.

Example 15 : S-1-(1,2,3,4-Tetrahydronaphthalen-1-yl)-2-mercapto-5-imidazolecarboxylic acid methyl ester - (compound 11.02).

a) S-N-(1,2,3,4-Tetrahydronaphthalen-1-yl)-glycine methyl ester.
The optically pure isomer of S-1-amino-1,2,3,4-tetrahydronaphthalene was prepared as described in US Patent No. 3,953,506
26.0 g of S-1-amino-1,2,3,4-tetrahydronaphthalene and 18.8 g of sodium carbonate are dispersed in 150 ml of methanol. 27.1 g of bromoacetic acid methyl ester are added dropwise to this dispersion. The mixture is stirred at room temperature for 100 hours. The precipitate is separated and the solution is concentrated to dryness, yielding the S-N-(1,2,3,4-tetrahydronaphthalen-1-yl) glycine methyl ester quantitatively.
b) S-N-Formyl-N-(1,2,3,4-tetrahydronaphthalen-1-yl)glycine methyl ester.
35.0 g of S-N-(1,2,3,4-tetrahydronaphthalen-1-yl)glycine methyl ester are added dropwise with cooling to 5° C to 72.3 ml of formic acid. Additionally 26.4 ml of acetic anhydride are added, and the mixture is kept at room temperature for 70 hours. Destillation under vacuum affords 20.4 g of S-N-formyl-N-(1,2,3,4-tetrahydronaphthalen-1-yl)glycine methyl ester having a melting point of 86-87° C.
c) A solution of 4.77 g of sodium methylate in 100 ml of tetrahydrofuran is prepared by adding suitable amounts of methanol and sodium hydride to the tetrahydrofuran. With optional cooling to room temperature 14.4 g of formic acid methyl ester and the S-N-formyl-N-(1,2,3,4-tetrahydronaphthalen-1-yl)-glycine methyl ester obtained from the process b) are added. After 20 hours the mixture is taken up with 55 ml of deionisized water and 110 ml of diethyl ether. The aqueous phase is separated, and 55 ml of methanol and 19.2 ml of 36 % hydrochloric acid are added. The solution is heated to 40°-50° C and treated with a solution of 12.9 g of potassium thiocyanate in 25 ml of deionisized water. The mixture is stirred at room temperature for 24 hours. During this period 9.5 8 of S-1-(1,2,3,4-tetrahydronaphthalen-1-yl)-2-mercapto-5-imidazolecarboxylic acid methyl ester precipitate. After recrystallisation from methanol the product has a melting point of 141-142° C, $[\alpha[_D^{20}$ = -87.9°.

Example 16 : S-1-(1,2,3,4-Tetrahydronaphthalen-1-yl)-5-imidazolecarboxylic acid methyl ester (compound 11.04).

0.5 g of sodium nitrite and 4.2 ml of nitric acid are solved in 50 ml of deionisized water. Within 1 hour 6.4 g of S-1-(1,2,3,4-tetrahydronaphthalen-1-yl)-2-mercapto-5-imidazolecarboxylic acid methyl ester are added portionwise at a temperature between 25° C and 30° C. The precipitate is isolated affording the nitric acid addition salt of S-1-(1,2,3,4-tetrahydronaphthalen-1-yl)-5-imidazolecarboxylic acid methyl ester. This salt is treated with 10 % aqueous sodium carbonate solution. Extracting the aqueous phase with chloroform, and evaporating the organic solvent yields 2.1 g of S-1-(1,2,3,4-tetrahydronaphthalen-1-yl)-5-imidazolecarboxylic acid methyl ester as a colourless resin, which is purified by chromatography through a silica column. The pure resinous product has a refraction index $[\alpha]_D^{20}$ = +23.6°.

All other compounds listed in the Tables 1, through 18 can be obtained by analogous methods of preparation.

## Claims

1. A method for controlling weeds, which method comprises applying to said weeds or to the locus thereof of a herbicidally effective amount of a 5-imidazolecarboxylic acid derivative of formula

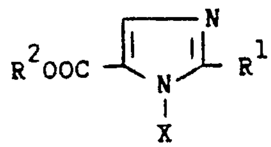

(I)

or a stereochemically isomeric form thereof, or of a salt thereof, wherein
$R^1$ is hydrogen or mercapto,
$R^2$ is hydrogen, $C_1$-$C_7$alkyl. $C_3$-$C_7$alkenyl, $C_3$-$C_7$alkynyl, $C_1$-$C_7$alkoxy-$C_1$-$C_7$alkyl, aryl-$C_1$-$C_5$alkyl or $C_3$-$C_7$cycloalkyl and
X is 1-indanyl, 1-tetrahydronaphthalenyl, 5-benzocycloheptanyl, 4-tetrahydrobenzothienyl, 4-tetrahydrobenzofuryl, 5-tetrahydroquinolyl. 5-tetrahydroisoquinolyl, 8-tetrahydroquinolyl, 8-tetrahydroisoquinolyl. 9,10-dihydro-9-antracenyl, 9H-fluoren-9-yl, 5-dibenzo[a,d]cycloheptenyl, 5-dibenzo[a,d]cycloheptanyl or 1-dihydronaphthalenyl each unsubstituted or substituted with one to six substituents selected from the group consisting of $C_1$-$C_5$alkyl, mono- and di(aryl) $C_1$-$C_5$alkyl, $C_1$-$C_5$alkoxy, halo, $C_3$-$C_7$alkenyl, amino, nitro, $C_1$-$C_6$alkylcarbonylamino, trifluoromethyl and difluoromethoxy, wherein two geminal substituents together with the carbon atom to which they are attached may form a $C_3$-$C_7$cycloalkyl group; wherein aryl is phenyl optionally substituted with one to three substituents each independently selected from the group consisting of $C_1$-$C_5$alkyl, $C_1$-$C_5$alkyloxy and halo.

2. A method according to claim 1, wherein
$R^2$ is hydrogen, $C_1$-$C_7$alkyl, $C_3$-$C_7$alkenyl, $C_3$-$C_7$alkynyl or $C_3$-$C_7$cycloalkyl, and
X is 1-indanyl, 1-tetrahydronaphthalenyl or 5-benzocycloheptanyl, each unsubstituted or substituted with up to six substituents selected from the group consisting of $C_1$-$C_5$alkyl, $C_1$-$C_5$alkoxy, halo, trifluoromethyl and difluoromethoxy.

3. A method according to claim 2, wherein X is a radical of formula

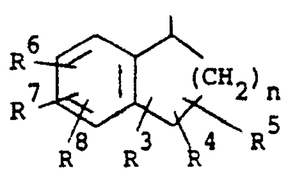

(a),

wherein $R^3$, $R^4$ and $R^5$ are each independently hydrogen or $C_1$-$C_5$alkyl,
$R^6$, $R^7$ and $R^8$ are each independently hydrogen, $C_1$-$C_5$alkyl, $C_1$-$C_5$alkoxy, halo, $CF_3$ or $OCHF_2$, and
n is 1 or 2 or 3.

4. A method according to claim 3, wherein n is one or two.

5. A method according to claim 4, wherein $R^3$, $R^4$ and $R^5$ are each independently hydrogen or methyl, $R^6$, $R^7$ and $R^8$ are each independently hydrogen, methyl, methoxy, fluoro, chloro or bromo, and $R^2$ is $C_1$-$C_4$alkyl.

6. A method according to claim 5, wherein $R^2$ is methyl.

7. A method according to claim 1, wherein the active ingredient is 1-(1,2,3,4-tetrahydronaphthalen-1-yl)-5-imidazolecarboxylic acid methyl ester.

8. A method according to claim 1, wherein the active ingredient is 1-(2,2-dimethyl-1,2,3,4-tetrahydronaphthalen-1-yl)-5-imidazolecarboxylic acid methyl ester.

9. A method according to any of claims 1-8, wherein the weeds are controlled in crops of useful plants.

10. A method according to claim 9, wherein the crop is rice.

11. A method according to claim 9, wherein the crop is maize.

12. A method according to claim 10, wherein the rice plants are transplanted rice plantlets.

13. A method according to claim 10 wherein 0.01 to 5.0 kg of the active ingredient is applied per hectare to areas where rice crops are grown.

14. A method according to claim 13, wherein 0.02 to 1.0 kg of the active ingredient is applied per hectare, after transplanting the rice plantlets.

15. A 5-imidazolecarboxylic acid derivative of formula

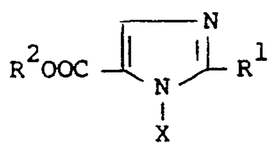

(I')

or a stereochemically isomeric form thereof, or a salt thereof,
wherein
$R^1$ is hydrogen or mercapto,
$R^2$ is hydrogen, $C_1$-$C_7$alkyl, $C_3$-$C_7$alkenyl, $C_3$-$C_7$alkynyl, $c_1$-$c_7$alkoxy-$c_1$-$C_7$alkyl, aryl-$C_1$-$C_5$alkyl or $C_3$-$C_7$cycloalkyl and
X is 1-indanyl, 1-tetrahydronaphthalenyl, 5-benzocycloheptanyl, 4-tetrahydrobenzothienyl, 4-tetrahydrobenzofuryl, 5-tetrahydroquinolyl, 5-tetrahydroisoquinolyl, 8-tetrahydroquinolyl, 8-tetrahydroisoquinolyl, 9,10-dihydro-9-anthracenyl, 9H-fluoren-9-yl, 5-dibenzo[a,d]cycloheptenyl, 5-dibenzo[a,d]cycloheptanyl or 1-dihydronaphthalenyl each unsubstituted or substituted with one to six substituents selected from the group consisting of $C_1$-$C_5$alkyl, mono- and di(aryl) $C_1$-$C_5$alkyl, $C_1$-$C_5$alkoxy, halo, $C_3$-$C_7$alkenyl, amino, nitro, $C_1$-$C_6$alkylcarbonylamino, trifluoromethyl and difluoromethoxy, wherein two geminal substituents together with the carbon atom to which they are attached may form a $C_3$-$C_7$cycloalkyl group; wherein aryl is phenyl optionally substituted with one to three substituents each independently selected from the group consisting of $C_1$-$C_5$alkyl, $C_1$-$C_5$alkyloxy and halo, provided that X is other than unsubstituted 1-indanyl, or unsubstituted 1-tetrahydronaphthalenyl.

16. A compound according to claim 15, wherein
$R^2$ is hydrogen, $C_1$-$C_7$alkyl, $C_3$-$C_7$alkenyl, $C_3$-$C_7$alkynyl or $C_3$-$C_7$cycloalkyl, and
X is 1-indanyl, 1-tetrahydronaphthalenyl or 5-benzocycloheptanyl, each unsubstituted or substituted with up to six substituents selected from the group consisting of $C_1$-$C_5$alkyl, $C_1$-$C_5$alkoxy, halo, trifluoromethyl and difluoromethoxy.

17. A compound according to claim 16, wherein X is a radical of formula

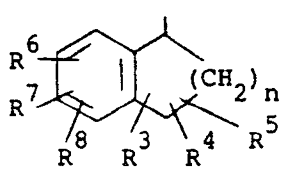

(a),

wherein
n is 1 or 2 or 3,
$R^3$, $R^4$ and $R^5$ are each independently hydrogen or $C_1$-$C_5$alkyl, $R^6$, $R^7$ and $R^8$ are each independently hydrogen, $C_1$-$C_5$alkyl, $C_1$-$C_5$alkoxy, halo, $CF_3$ or $OCHF_2$.

18. A compound according to claim 17, wherein n is one or two, $R^3$, $R^4$ and $R^5$ are each independently

hydrogen or methyl, $R^6$ , $R^7$ and $R^8$ are each independently hydrogen, methyl, methoxy, fluoro, chloro or bromo, and $R^2$ is $C_1$-$C_4$alkyl.

19. A compound according to claim 18, wherein $R^2$ is methyl.

20. A compound according to claim 15, wherein the compound of formula (I') is 1-(2,2-dimethyl-1,2,3,4-tetrahydronaphthalen-1-yl)-5-imidazolecarboxylic acid methyl ester.

21. A herbicidal composition, containing inert carriers and, if desired, other adjuvants, and as active ingredient an herbicidally effective amount of a 5-imidazolecarboxylic acid derivative as claimed in claim 15.

22. A composition according to claim 21, wherein
$R^1$ is hydrogen or mercapto,
$R^2$ is hydrogen, $C_1$-$C_7$alkyl, $C_3$-$C_7$alkenyl, $C_3$-$C_7$alkynyl or $C_3$-$C_7$cycloalkyl, and
X is 1-indanyl, 1-tetrahydronaphthalenyl or 5-benzocycloheptanyl, each unsubstituted or substituted with up to six substituents selected from the group consisting of $C_1$-$C_5$alkyl, $C_1$-$C_5$alkoxy, halo, trifluoromethyl and difluoromethoxy.

23. A composition according to claim 22 wherein X is a radical of formula

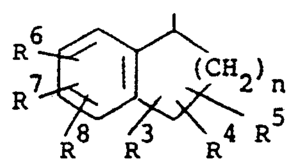

(a) ,

wherein $R^3$, $R^4$ and $R^5$ are each independently hydrogen or $C_1$-$C_5$alkyl, $R^6$, $R^7$ and $R^8$ are each independently hydrogen, $C_1$-$C_5$alkyl, $C_1$-$C_5$ alkoxy, halo, $CF_3$ or $OCHF_2$, and
n is 1 or 2 or 3.

24. A method of preparing a herbicidal composition according to any of claims 21 to 23, characterized in that a herbicidally effective amount of a compound as defined in any of claims 21 to 23 is intimately mixed with suitable carriers and, if desired, with other adjuvants.

25. A process for preparing a chemical compound as claimed in any one of claims 15 to 20, characterized by condensing a compound of formula

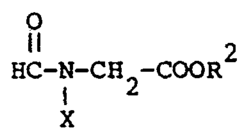

(II)

wherein $R^2$ and X are as defined hereinabove, with a $C_1$-$C_4$alkyl ester of formic acid in the presence of base and in a reaction-inert solvent; and treating the resulting intermediate of formula

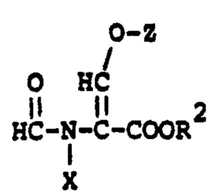

(III)

wherein $R^2$ and X are as defined hereinabove and Z is an alkali metal atom, either
a) with an alkali metal isothiocyanate in the presence of an acid, thus obtaining a 2-mercap-

toimidazole compound of formula

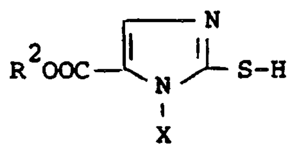

(Ia)

wherein $R^2$ and X are as defined hereinabove, which optionally is converted into a compound of formula

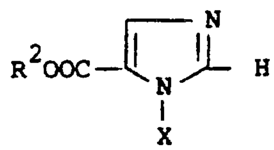

(Ib)

by reaction with sodium nitrite in the presence of nitric acid in water; or by treatment with Raney-nickel in the presence of a lower aliphatic alcohol, at a temperature between 40° C and 80° C; or by treatment with an aqueous $H_2O_2$ solution preferably in the presence of a carboxylic acid; or
b) with a carboxylic acid amide of 1 to 3 carbon atoms, preferably formamide, in the presence of an acid at a temperature between 50° C and 250° C; or
c) with an excess of ammonium carbonate or hydrogen carbonate in a suitable solvent, eighter an inert solvent or an acid, at a temperature between 20° C and 20° C; and optionally converting the compounds of formula (I') into the salt-form by treatment with an acid or a base and preparing stereochemically isomeric forms thereof.

## Revendications

1. Procédé pour maîtriser des mauvaises herbes, lequel procédé comprend l'application auxdites mauvaises herbes ou à leur site d'une quantité herbicide efficace d'un dérivé d'acide 5-imidazolecarboxylique de formule

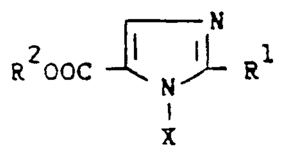

(I)

ou d'une forme isomère stéréochimique de celui-ci, ou d'un sel de celui-ci, dans laquelle
$R^1$ est un hydrogène ou un mercapto,
$R^2$ est un hydrogène, un alkyle en $C_1$-$C_7$, un alcényle en $C_3$-$C_7$, un alcynyle en $C_3$-$C_7$, un (alcoxy en $C_1$-$C_7$)alkyle en $C_1$-$C_7$, un arylalkyle en $C_1$-$C_5$ ou un cycloalkyle en $C_3$-$C_7$ et
X est un 1-indanyle, un 1-tétrahydronaphtalényle, un 5-benzocycloheptanyle, un 4-tétrahydrobenzothiényle, un 4-tétrahydrobenzofuryle, un 5-tétrahydroquinolyle, un 5-tétrahydro-isoquinolyle, un 8-tétrahydroquinolyle, un 8-tétrahydro-isoquinolyle, un 9,10-dihydro-9-anthracényle, un 9H-fluorène-9-yle, un 5-dibenzo[a,d]cycloheptényle, un 5-dibenzo[a,d]cycloheptanyle ou un 1-dihydronaphtalényle chacun non substitué ou substitué par un à six substituants choisis dans le groupe constitué par un alkyle en $C_1$-$C_5$, un mono- ou di(aryl)alkyle en $C_1$-$C_5$, un alcoxy en $C_1$-$C_5$, un halogéno, un alcényle en $C_3$-$C_7$, un amino, un nitro, un (alkyl en $C_1$-$C_6$)carbonylamino, un trifluorométhyle et un difluorométhoxy, où deux substituants géminés, avec l'atome de carbone auquel ils sont fixés, peuvent former un groupe cycloalkyle en $C_3$-$C_7$ ;
où un aryle est un phényle facultativement substitué par un à trois substituants choisis chacun indépendamment dans le groupe constitué par un alkyle en $C_1$-$C_5$, un alkyloxy en $C_1$-$C_5$ et un halogéno.

**2.** Procédé selon la revendication 1, dans lequel $R^2$ est un hydrogène, un alkyle en $C_1$-$C_7$, un alcényle en $C_3$-$C_7$, un alcynyle en $C_3$-$C_7$ ou un cycloalkyle en $C_3$-$C_7$, et
X est un 1-indanyle, un 1-tétrahydronaphtalényle ou un 5-benzocycloheptanyle, chacun non substitué ou substitué par jusqu'à six substituants choisis dans le groupe constitué par un alkyle en $C_1$-$C_5$, un alcoxy en $C_1$-$C_5$, un halogéno, un trifluorométhyle et un difluorométhoxy.

**3.** Procédé selon la revendication 2, dans lequel X est un radical de formule

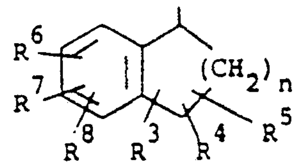

(a),

dans laquelle $R^3$, $R^4$ et $R^5$ sont chacun indépendamment un hydrogène ou un alkyle en $C_1$-$C_5$,
$R^6$, $R^7$ et $R^8$ sont chacun indépendamment un hydrogène, un alkyle en $C_1$-$C_5$, un alcoxy en $C_1$-$C_5$, un halogéno, $CF_3$ ou $OCHF_2$, et n est 1, 2 ou 3.

**4.** Procédé selon la revendication 3, dans lequel n est un ou deux.

**5.** Procédé selon la revendication 4, dans lequel $R^3$, $R^4$ et $R^5$ sont chacun indépendamment un hydrogène ou un méthyle, $R^6$, $R^7$ et $R^8$ sont chacun indépendamment un hydrogène, un méthyle, un méthoxy, un fluoro, un chloro ou un bromo, et $R^2$ est un alkyle en $C_1$-$C_4$.

**6.** Procédé selon la revendication 5, dans lequel $R^2$ est un méthyle.

**7.** Procédé selon la revendication 1, dans lequel l'ingrédient actif est l'ester méthylique de l'acide 1-(1,2,3,4-tétrahydronaphtalène-1-yl)-5-imidazolecarboxylique.

**8.** Procédé selon la revendication 1, dans lequel l'ingrédient actif est l'ester méthylique de l'acide 1-(2,2-diméthyl-1,2,3,4-tétrahydronaphtalène-1-yl)-5-imidazolecarboxylique.

**9.** Procédé selon l'une quelconque des revendications 1 à 8, dans lequel les mauvaises herbes sont maîtrisées dans des cultures de plantes utiles.

**10.** Procédé selon la revendication 9, dans lequel la culture est une culture de riz.

**11.** Procédé selon la revendication 9, dans lequel la culture est une culture de maïs.

**12.** Procédé selon la revendication 10, dans lequel le riz est constitué de jeunes plants de riz repiqués.

**13.** Procédé selon la revendication 10, dans lequel on applique 0,01 à 5,0 kg de l'ingrédient actif par hectare aux zones où l'on cultive du riz.

**14.** Procédé selon la revendication 13, dans lequel on applique 0,02 à 1,0 kg de l'ingrédient actif par hectare après repiquage des jeunes plants de riz.

**15.** Dérivé d'acide 5-imidazolecarboxylique de formule

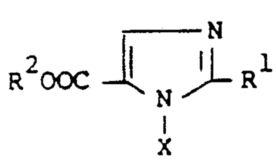

(I')

ou d'une forme isomère stéréochimique de celui-ci, ou d'un sel de celui-ci, dans laquelle
$R^1$ est un hydrogène ou un mercapto,

$R^2$ est un hydrogène, un alkyle en $C_1$-$C_7$, un alcényle en $C_3$-$C_7$, un alcynyle en $C_3$-$C_7$ , un (alcoxy en $C_1$-$C_7$)alkyle en $C_1$ -$C_7$, un arylalkyle en $C_1$ -$C_5$ ou un cycloalkyle en $C_3$-$C_7$ et
X est un 1-indanyle, un 1-tétrahydronaphtalényle, un 5-benzocycloheptanyle, un 4-tétrahydrobenzothiényle, un 4-tétrahydrobenzofuryle, un 5-tétrahydroquinolyle, un 5-tétrahydro-isoquinolyle, un 8-tétrahydroquinolyle, un 8-tétrahydro-isoquinolyle, un 9,10-dihydro-9-anthracényle, un 9H-fluorène-9-yle, un 5-dibenzo[a,d]cycloheptényle, un 5-dibenzo[a,d]cycloheptanyle ou un 1-dihydronaphtalényle chacun non substitué ou substitué par un à six substituants choisis dans le groupe constitué par un alkyle en $C_1$-$C_5$, un mono- ou di(aryl)alkyle en $C_1$-$C_5$, un alcoxy en $C_1$-$C_5$, un halogéno, un alcényle en $C_3$-$C_7$, un amino, un nitro, un (alkyl en $C_1$-$C_6$)carbonylamino, un trifluorométhyle et un difluorométhoxy, où deux substituants géminés, avec l'atome de carbone auquel ils sont fixés, peuvent former un groupe cycloalkyle en $C_3$-$C_7$ ;
où un aryle est un phényle facultativement substitué par un à trois substituants choisis chacun indépendamment dans le groupe constitué par un alkyle en $C_1$-$C_5$, un alkyloxy en $C_1$-$C_5$ et un halogéno, sous réserve que X soit autre qu'un 1-indanyle non substitué ou un 1-tétrahydronaphtalényle non substitué.

16. Composé selon la revendication 15, dans lequel $R^2$ est un hydrogène, un alkyle en $C_1$-$C_7$, un alcényle en $C_3$-$C_7$, un alcynyle en $C_3$-$C_7$ ou un cycloalkyle en $C_3$-$C_7$, et
X est un 1-indanyle, un 1-tétrahydronaphtalényle ou un 5-benzocycloheptanyle, chacun non substitué ou substitué par jusqu'à six substituants choisis dans le groupe constitué par un alkyle en $C_1$ -$C_5$, un alcoxy en $C_1$-$C_5$, un halogéno, un trifluorométhyle et un difluorométhoxy.

17. Composé selon la revendication 16, dans lequel X est un radical de formule

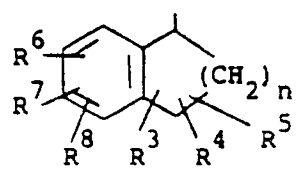

(a),

dans laquelle
n est 1, 2 ou 3,
$R^3$, $R^4$ et $R^5$ sont chacun indépendamment un hydrogène ou un alkyle en $C_1$-$C_5$, et
$R^6$, $R^7$ et $R^8$ sont chacun indépendamment un hydrogène, un alkyle en $C_1$-$C_5$, un alcoxy en $C_1$-$C_5$, un halogéno, $CF_3$ ou $OCHF_2$.

18. Composé selon la revendication 17, dans lequel n est un ou deux , $R^3$ , $R^4$ et $R^5$ sont chacun indépendamment un hydrogène ou un méthyle, $R^6$, $R^7$ et $R^8$ sont chacun indépendamment un hydrogène, un méthyle, un méthoxy, un fluoro, un chloro ou un bromo, et $R^2$ est un alkyle en $C_1$-$C_4$.

19. Composé selon la revendication 18, dans lequel $R^2$ est un méthyle.

20. Composé selon la revendication 15, dans lequel le composé de formule (I') est l'ester méthylique de l'acide 1-(2,2-diméthyl-1,2,3,4-tétrahydronaphtalène-1-yl)-5-imidazolecarboxylique.

21. Composition herbicide contenant des supports inertes et, le cas échéant, d'autres adjuvants et, comme ingrédient actif, une quantité herbicide efficace d'un dérivé d'acide 5-imidazolecarboxylique selon la revendication 15.

22. Composition herbicide selon la revendication 21, dans laquelle
$R^1$ est un hydrogène ou un mercapto,
$R^2$ est un hydrogène, un alkyle en $C_1$-$C_7$, un alcényle en $C_3$-$C_7$, un alcynyle en $C_3$-$C_7$ ou un cycloalkyle en $C_3$-$C_7$, et
X est un 1-indanyle, un 1-tétrahydronaphtalényle ou un 2-benzocycloheptanyle, chacun substitué ou non substitué avec jusqu'à six substituants choisis dans le groupe constitué par un alkyle en $C_1$-$C_5$, un alcoxy en $C_1$-$C_5$, un halogéno, un trifluorométhyle et un difluorométhoxy.

**23.** Composition selon la revendication 22, dans laquelle X est un radical de formule

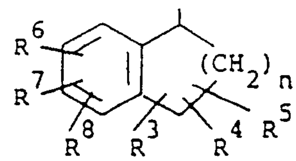

(a),

dans laquelle $R^3$, $R^4$ et $R^5$ sont chacun indépendamment un hydrogène ou un alkyle en $C_1$-$C_5$,
$R^6$, $R^7$ et $R^8$ sont chacun indépendamment un hydrogène, un alkyle en $C_1$-$C_5$, un alcoxy en $C_1$-$C_5$, un halogéno, $CF_3$ ou $OCHF_2$, et
n est 1, 2 ou 3.

**24.** Procédé pour préparer une composition herbicide selon l'une quelconque des revendications 21 à 23, caractérisé en ce que l'on mélange intimement une quantité herbicide efficace d'un composé comme défini dans l'une quelconque des revendications 21 à 23 avec des supports appropriés et, le cas échéant, d'autres adjuvants.

**25.** Procédé pour préparer un composé chimique selon l'une quelconque des revendications 15 à 20, caractérisé par la condensation d'un composé de formule

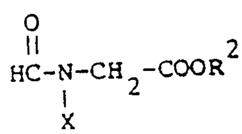

(II)

dans laquelle $R^2$ et X sont comme précédemmment défini, avec un ester d'alkyle en $C_1$-$C_4$ de l'acide formique en présence d'une base et dans un solvant inerte vis-à-vis de la réaction ; et le traitement de l'intermédiaire obtenu de formule

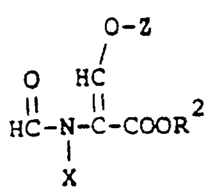

(III)

dans laquelle $R^2$ et X sont comme précédemment défini et Z est un atome de métal alcalin, avec soit
a) un isothiocyanate de métal alcalin en présence d'un acide pour obtenir un 2-mercapto-imidazole de formule

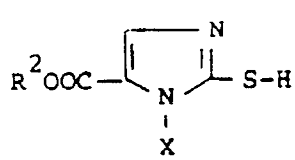

(Ia)

dans laquelle $R^2$ et X sont comme précédemment défini, qui facultativement est transformé en un composé de formule

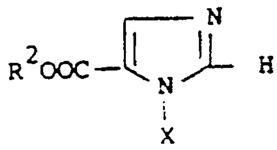

(Ib)

par réaction avec le nitrite de sodium en présence d'acide nitrique dans l'eau ; ou par traitement avec du nickel de Raney en présence d'un alcool aliphatique inférieur à une température entre 40 °C et 80 °C ; ou par traitement avec une solution aqueuse d'$H_2O_2$, de préférence en présence d'un acide carboxylique ; soit

b) un amide d'acide carboxylique de 1 à 3 atomes de carbone, de préférence le formamide, en présence d'un acide à une température entre 50 °C et 250 °C; soit

c) un excès de carbonate ou d'hydrogénocarbonate d'ammonium dans un solvant approprié qui est soit un solvant inerte, soit un acide, à une température entre 20 °C et 200 °C ; et facultativement la transformation des composés de formule (I') en un sel par traitement avec un acide ou une base et la préparation de leurs formes isomères stéréochimiques.

**Patentansprüche**

1. Ein Verfahren zur Bekämpfung von Unkräutern, welches Verfahren ein Aufbringen einer herbizid wirksamen Menge eines 5-Imidazolcarbonsäurederivats der Formel

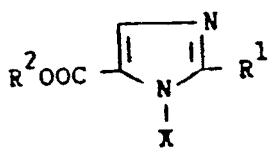

(I)

oder einer stereochemisch isomeren Form hievon oder eines Salzes hievon auf diese Unkräuter oder auf deren Wachstumsort umfaßt, in welcher Formel
$R^1$ für Wasserstoff oder Mercapto steht,
$R^2$ für Wasserstoff, $C_1$-$C_7$Alkyl, $C_3$-$C_7$Alkenyl, $C_3$-$C_7$Alkinyl, $C_1$-$C_7$-Alkoxy-$C_1$-$C_7$alkyl, Aryl-$C_1$-$C_5$alkyl oder $C_3$-$C_7$Cycloalkyl steht und
X 1-Indanyl, 1-Tetrahydronaphthyl, 5-Benzocycloheptanyl, 4-Tetrahydrobenzothienyl, 4-Tetrahydrobenzofuryl, 5-Tetrahydrochinolyl, 5-Tetrahydroisochinolyl, 8-Tetrahydrochinolyl, 8-Tetrahydroisochinolyl, 9,10-Dihydro-9-anthracenyl, 9H-Fluoren-9-yl, 5-Dibenzo [a,d]cycloheptenyl, 5-Dibenzo[a,d]cycloheptanyl oder 1-Dihydronaphthyl darstellt, jeweils unsubstituiert oder substituiert mit einem bis 6 Substituenten, ausgewählt aus der aus $C_1$-$C_5$Alkyl, Mono- und Di( aryl)-$C_1$-$C_5$alkyl, $C_1$-$C_5$Alkoxy, Halogen, $C_3$-$C_7$Alkenyl, Amino, Nitro, $C_1$-$C_6$Alkylcarbonylamino, Trifluormethyl und Difluormethoxy bestehenden Gruppe, worin zwei geminale Substituenten, zusammen mit dem Kohlenstoffatom, an das sie gebunden sind, eine $C_3$-$C_7$Cycloalkylgruppe ausbilden können; wobei Aryl Phenyl bedeutet, das gegebenenfalls mit einem bis drei Substituenten, jeweils unabhängig voneinander ausgewählt aus der aus $C_1$-$C_5$Alkyl, $C_1$-$C_5$Alkyloxy und Halogen bestehenden Gruppe, substituiert sein kann.

2. Verfahren nach Anspruch 1, worin
$R^2$ für Wasserstoff, $C_1$-$C_7$Alkyl, $C_3$-$C_7$Alkenyl, $C_3$-$C_7$Alkinyl oder $C_3$-$C_7$Cycloalkyl steht und
X 1-Indanyl, 1-Tetrahydronaphthyl oder 5-Benzocycloheptanyl darstellt, jeweils unsubstituiert oder substituiert mit bis zu 6 Substituenten, ausgewählt aus der aus $C_1$-$C_5$Alkyl, $C_1$-$C_5$Alkoxy, Halogen, Trifluormethyl und Difluormethoxy bestehenden Gruppe.

3. Verfahren nach Anspruch 2, worin X einen Rest der Formel

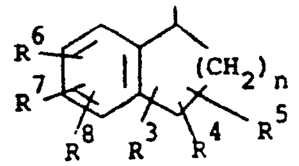

(a)

bedeutet, worin
$R^3$, $R^4$ und $R^5$ jeweils unabhängig voneinander Wasserstoff oder $C_1$-$C_5$Alkyl bedeuten,
$R^6$, $R^7$ und $R^8$ unabhängig voneinander für Wasserstoff, $C_1$-$C_5$Alkyl, $C_1$-$C_5$Alkoxy, Halogen, $CF_3$ oder $OCHF_2$ stehen und
n den Wert 1 oder 2 oder 3 aufweist.

**4.** Verfahren nach Anspruch 3, worin n den Wert 1 oder 2 aufweist.

**5.** Verfahren nach Anspruch 4, worin
$R^3$, $R^4$ und $R^5$ jeweils unabhängig voneinander Wasserstoff oder Methyl bedeuten,
$R^6$, $R^7$ und $R^8$ jeweils unabhängig voneinander für Wasserstoff, Methyl, Methoxy, Fluor, Chlor oder Brom stehen und
$R^2$ $C_1$-$C_4$Alkyl darstellt.

**6.** Verfahren nach Anspruch 5, worin $R^2$ für Methyl steht.

**7.** Verfahren nach Anspruch 1, worin der wirksame Bestandteil 1-(1,2,3,4-Tetrhydronaphth-1-yl)-5-imidazolcarbonsäuremethylester ist.

**8.** Verfahren nach Anspruch 1, worin der wirksame Bestandteil 1-(2,2-Dimethyl-1,2,3,4-tetrahydronaphth-1-yl)-5-imidazolcarbonsäuremethylester ist.

**9.** Verfahren nach einem der Ansprüche 1 bis 8, worin die Unkräuter in Anpflanzungen von Nutzpflanzen bekämpft werden.

**10.** Verfahren nach Anspruch 9, worin die Nutzpflanzung Reis ist.

**11.** Verfahren nach Anspruch 9, worin die Nutzpflanzung Mais ist.

**12.** Verfahren nach Anspruch 10, worin die Reispflanzen ausgepflanzte Reispflänzchen sind.

**13.** verfahren nach Anspruch 10, worin 0,01 bis 5,0 kg wirksamer Bestandteil je ha Fläche aufgewendet wird, auf der Reispflanzen gezogen werden.

**14.** Verfahren nach Anspruch 13, worin 0,02 bis 1,0 kg wirksamer Bestandteil je ha nach dem Auspflanzen der Reispflänzchen angewendet werden.

**15.** Ein 5-Imidazolcarbonsäurederviat der Formel

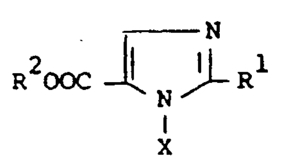

(I')

oder eine stereochemisch isomere Form hievon oder ein Salz hievon, worin
$R^1$ für Wasserstoff oder Mercapto steht,
$R^2$ für Wasserstoff, $C_1$-$C_7$Alkyl, $C_3$-$C_7$Alkenyl, $C_3$-$C_7$Alkinyl, $C_1$-$C_7$-Alkoxy-$C_1$-$C_7$alkyl, Aryl-$C_1$-$C_5$alkyl oder $C_3$-$C_7$Cycloalkyl steht und
X 1-Indanyl, 1-Tetrahydronaphthyl, 5-Benzocycloheptanyl, 4-Tetrahydrobenzothienyl, 4-Tetrahydrobenzofuryl, 5-Tetrahydrochinolyl, 5-Tetrahydroisochinolyl, 8-Tetrahydrochinolyl, 8-Tetrahydroisochinolyl,

9,10-Dihydro-9-anthracenyl, 9H-Fluoren-9-yl, 5-Dibenzo [a,d]cycloheptenyl, 5-Dibenzo[a,d]cycloheptanyl oder 1-Dihydronaphthyl darstellt, jeweils unsubstituiert oder substituiert mit einem bis 6 Substituenten, ausgewählt aus der aus $C_1$-$C_5$Alkyl, Mono- und Di( aryl)-$C_1$-$C_5$alkyl, $C_1$-$C_5$Alkoxy, Halogen, $C_3$-$C_7$Alkenyl, Amino, Nitro, $C_1$-$C_6$Alkylcarbonylamino, Trifluormethyl und Difluormethoxy bestehenden Gruppe, worin zwei geminale Substituenten, zusammen mit dem Kohlenstoffatom, an das sie gebunden sind, eine $C_3$-$C_7$Cycloalkylgruppe ausbilden können; wobei Aryl Phenyl bedeutet, das gegebenenfalls mit einem bis drei Substituenten, jeweils unabhängig voneinander ausgewählt aus der aus $C_1$-$C_5$Alkyl, $C_1$-$C_5$Alkyloxy und Halogen bestehenden Gruppe, substituiert sein kann, mit der Maßgabe, daß X eine andere Bedeutung als unsubstituiertes 1-Indanyl oder unsubstituiertes 1-Tetrahydronaphthyl aufweist.

**16.** Verbindung nach Anspruch 15, worin
$R^2$ für Wasserstoff, $C_1$-$C_7$Alkyl, $C_3$-$C_7$Alkenyl, $C_3$-$C_7$Alkinyl oder $C_3$-$C_7$-Cycloalkyl steht und
X 1-Indanyl, 1-Tetrahydronaphthyl oder 5-Benzocycloheptanyl bedeutet, jeweils unsubstituiert oder substituiert mit bis zu 6 Substituenten, ausgewählt aus der aus $C_1$-$C_5$Alkyl, $C_1$-$C_5$Alkoxy, Halogen, Trifluormethyl und Difluormethoxy bestehenden Gruppe.

**17.** Verbindung nach Anspruch 16, worin X einen Rest der Formel

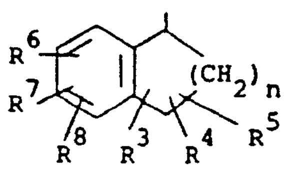

(a)

darstellt, worin
n den Wert 1 oder 2 oder 3 aufweist,
$R^3$, $R^4$ und $R^5$ jeweils unabhängig voneinander für Wasserstoff oder $C_1$-$C_5$Alkyl stehen und
$R^6$, $R^7$ und $R^8$ jeweils unabhängig voneinander Wasserstoff, $C_1$-$C_5$Alkyl, $C_1$-$C_5$Alkoxy, Halogen, $CF_3$ oder $OCHF_2$ bedeuten.

**18.** Verbindung nach Anspruch 17, worin
n den Wert 1 oder 2 aufweist,
$R^3$, $R^4$ und $R^5$ jeweils unabhängig voneinander Wasserstoff oder Methyl bedeuten,
$R^6$, $R^7$ und $R^8$ jeweils unabhängig voneinander für Wasserstoff, Methyl, Methoxy, Fluor, Chlor oder Brom stehen und
$R^2$ $C_1$-$C_4$Alkyl darstellt.

**19.** Verbindung nach Anspruch 18, worin $R^2$ Metyl bedeutet.

**20.** Verbindung nach Anspruch 15, worin die Verbindung der Formel (I') 1-(2,2-Dimethyl-1,2,3,4-tetrahydronaphth-1-yl)-5-imidazolcarbonsäuremethylester ist.

**21.** Eine herbizide Zusammensetzung, enthaltend inerte Träger und gewünschtenfalls weitere Hilfsmittel, und als wirksamen Bestandteil eine herbizid wirksame Menge eines 5-Imidazolcarbonsäurederivats, wie in Anspruch 15 beansprucht.

**22.** Zusammensetzung nach Anspruch 21, worin
$R^1$ für Wasserstoff oder Mercapto steht,
$R^2$ Wasserstoff, $C_1$-$C_7$Alkyl, $C_3$-$C_7$Alkenyl, $C_3$-$C_7$Alkinyl oder $C_3$-$C_7$Cycloalkyl darstellt und
X 1-Indanyl, 1-Tetrahydronaphthyl oder 5-Benzocycloheptanyl bedeutet, jeweils unsubstituiert oder substituiert mit bis zu 6 Substituenten, ausgewählt aus der aus $C_1$-$C_5$Alkyl, $C_1$-$C_5$Alkoxy, Halogen, Trifluormethyl und Difluormethoxy bestehenden Gruppe.

**23.** Zusammensetzung nach Anspruch 22, worin X einen Rest der Formel

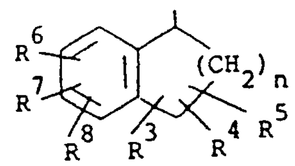

(a)

bedeutet, worin
$R^3$, $R^4$ und $R^5$ jeweils unabhängig voneinander Wasserstoff oder $C_1$-$C_5$Alkyl bedeuten,
$R^6$, $R^7$ und $R^8$ unabhängig voneinander für Wasserstoff,$C_1$-$C_5$Alkyl, $C_1$-$C_5$Alkoxy, Halogen, $CF_3$ oder $OCHF_2$ stehen und
n den Wert 1 oder 2 oder 3 aufweist.

**24.** Verfahren zur Herstellung einer herbiziden Zusammensetzung nach einem der Ansprüche 21 bis 23, dadurch gekennzeichnet, daß eine herbizid wirksame Menge einer Verbindung, wie in einem der Ansprüche 21 bis 23 definiert, innig mit geeigneten Trägern und gewünschtenfalls mit weiteren Hilfsstoffen vermischt wird.

**25.** Verfahren zur Herstellung einer chemischen Verbindung, wie in einem der Ansprüche 15 bis 20 beansprucht, gekennzeichnet durch Kondensieren einer Verbindung der Formel

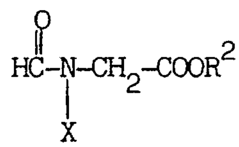

(II),

worin $R^2$ und X wie oben definiert sind, mit einem $C_1$-$C_4$Alkylester der Ameisensäure in Anwesenheit einer Base in einem reaktionsinerten Lösungsmittel; und Behandeln des gebildeten Zwischenproduktes der Formel

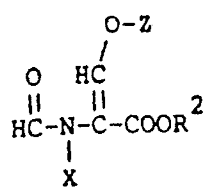

(III),

worin
$R^2$ und X wie oben definiert sind und
Z ein Alkalimetallatom bedeutet, entweder
a) mit einem Alkalimetallisothiocyanat in Anwesenheit einer Säure, unter Ausbildung einer 2-Mercaptoimidazolverbindung der Formel

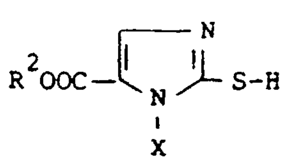

(Ia),

worin
$R^2$ und X wie oben definiert sind, die gewünschtenfalls zu einer Ver bindung der Formel

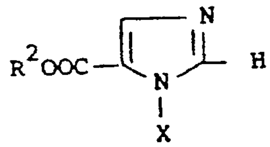

(Ib)

durch Umsetzung mit Natriumnitrit in Anwesenheit von Salpetersäure in Wasser; oder durch Behandlung mit Raney-Nickel in Anwesenheit eines niederen aliphatischen Alkohols bei einer Temperatur zwischen 40 und 80 °C; oder durch Behandlung mit einer wäßrigen $H_2O_2$-Lösung, vorzugsweise in Anwesenheit einer Carbonsäure, umgewandelt wird; oder

b) mit einem Carbonsäureamid mit 1 bis 3 Kohlenstoffatomen, vorzugsweise Formamid, in Anwesenheit einer Säure bei einer Temperatur zwischen 50 °C und 250 °C; oder

c) mit einem Überschuß an Ammoniumcarbonat oder -hydrogencarbonat in einem geeigneten Lösungsmittel, entweder einem inerten Lösungsmittel oder einer Säure,bei einer Temperatur zwischen 20 °C und 200 °C; und gewünschtenfalls Überführen der Verbindungen der Formel (I') in die Salzform durch Behandeln mit einer Säure oder einer Base und Herstellen stereochemisch isomerer Formen hievon.